# EUROPEAN PATENT SPECIFICATION

(11) **EP 2 071 039 B1**
(45) Date of publication and mention of the grant of the patent: **27.05.2015**
(21) Application number: 07301664.4
(22) Date of filing: 12.12.2007
(51) Int. Cl.: C12Q 1/68

(54) **Method for testing a subject thought to be predisposed to lung cancer**
Verfahren zur Überprüfung einer Person mit Verdacht auf Prädisposition für Lungenkrebs
Procédé pour tester un sujet prédisposé à un cancer des poumons

(43) Date of publication of application: 17.06.2009
(73) Proprietor: Commissariat à l'Énergie Atomique et aux Énergies Alternatives, 75015 Paris (FR)
(72) Inventor: Brennan, Paul, 69001 Lyon (FR); Gut, Yvo, Glynne, 75014 Paris (FR); Heath, Simon, 75010 Paris (FR); Lathrop, Mark, 75012 Paris (FR)
(74) Representative: Regimbeau

(56) References cited:
- WO-A-2006/123955
- SACCONE SCOTT F ET AL: "Cholinergic nicotinic receptor genes implicated in a nicotine dependence association study targeting 348 candidate genes with 3713 SNPs." HUMAN MOLECULAR GENETICS 1 JAN 2007, vol. 16, no. 1, 1 January 2007 (2007-01-01), pages 36-49, XP002482274 ISSN: 0964-6906
- DATABASE BIOSIS [Online] BIOSCIENCES INFORMATION SERVICE, PHILADELPHIA, PA, US; February 1998 (1998-02), KUNZE U ET AL: "Preventive oncology and nicotine addition: First results of a field study among lung cancer patients" XP002482278 Database accession no. PREV199800305829 & ATEMWEGS- UND LUNGENKRANKHEITEN, vol. 24, no. SUPPL. 1, February 1998 (1998-02), pages S95-S96, ISSN: 0341-3055
- AMOS CHRISTOPHER I ET AL: "Genome-wide association scan of tag SNPs identifies a susceptibility locus for lung cancer at 15q25.1." NATURE GENETICS MAY 2008, vol. 40, no. 5, May 2008 (2008-05), pages 616-622, XP009100741 ISSN: 1546-1718
- HUNG RAYJEAN J ET AL: "A susceptibility locus for lung cancer maps to nicotinic acetylcholine receptor subunit genes on 15q25" NATURE (LONDON), vol. 452, no. 7187, April 2008 (2008-04), pages 633-637, XP002482276 ISSN: 0028-0836
- THORGEIRSSON THORGEIR E ET AL: "A variant associated with nicotine dependence, lung cancer and peripheral arterial disease" NATURE (LONDON), vol. 452, no. 7187, April 2008 (2008-04), page 638, XP002482277 ISSN: 0028-0836
- ANONYMOUS: 'Reference SNP(refSNP) Cluster Report: rs17487223', [Online] 13 September 2004, Reference SNP Retrieved from the Internet: <URL:http://www.ncbi.nlm.nih.gov/projects/S NP/snp_ref.cgi?rs=17487223> [retrieved on 2011-04-28]
- ANONYMOUS: 'Genecards report for CHRNB4', [Online] 31 December 2010, Genecards Retrieved from the Internet: <URL:http://www.genecards.org/cgi-bin/cardd isp.pl?gene=CHRNB4&search=chrna3> [retrieved on 2011-04-28]
- MA JENNIE Z ET AL: "Haplotype analysis indicates an association between the DOPA decarboxylase (DDC) gene and nicotine dependence", HUMAN MOLECULAR GENETICS, vol. 14, no. 12, June 2005 (2005-06), pages 1691-1698,
- BEUTEN JOKE ET AL: "Single- and multilocus allelic variants within the GABA(B) receptor subunit 2 (GABAB2) gene are significantly associated with nicotine dependence", AMERICAN JOURNAL OF HUMAN GENETICS, vol. 76, no. 5, May 2005 (2005-05), pages 859-864,
- BIROS E ET AL: "Germ line polymorphisms of the tumor suppressor gene p53 and lung cancer.", LUNG CANCER (AMSTERDAM, NETHERLANDS) 2001 FEB-MAR, vol. 31, no. 2-3, February 2001 (2001-02), pages 157-162, ISSN: 0169-5002
- SAKIYAMA T ET AL: "Association of amino acid substitution polymorphisms in DNA repair genes TP53, POLI, REV1 and LIG4 with lung cancer risk", INTERNATIONAL JOURNAL OF CANCER, UNION INTERNATIONALE CENTRE LE CANCER, vol. 114, 1 January 2005 (2005-01-01), pages 730-737, XP002997499, ISSN: 0020-7136, DOI: 10.1002/IJC.20790

### SEQUENCE LISTING

<110> Commissariat à l'énergie atomique
   Centre International de Recherche sur le Cancer
<120> METHOD FOR TESTING A SUBJECT THOUGHT TO BE PREDISPOSED TO LUNG CANCER
<130> 67292
<160> 64
<170> PatentIn version 3.3
<210> 1
   <211> 31
   <212> DNA
   <213> Homo sapiens
<220>
   <221> variation
   <222> (16)..(16)
   <223> n= G or A
<400> 1
   caatagaaaa cttaangccg caactaagta a 31
<210> 2
   <211> 31
   <212> DNA
   <213> Homo sapiens
<220>
   <221> variation
   <222> (16)..(16)
   <223> n= C or T
<400> 2
   ctaaggcccc tcagtntctc tgtgcctctg g 31
<210> 3
   <211> 31
   <212> DNA
   <213> Homo sapiens
<220>
   <221> variation
   <222> (16)..(16)
   <223> n= T or C
<400> 3
   atggtgagtt tgcatnaatt ttgaaaagaa a 31
<210> 4
   <211> 31
   <212> DNA
   <213> Homo sapiens
<220>
   <221> variation
   <222> (16)..(16)
   <223> n= T or G
<400> 4
   ctagagtcaa gtaagngttt ctgtctctaa c 31
<210> 5
   <211> 31
   <212> DNA
   <213> Homo sapiens
<220>
   <221> variation
   <222> (16)..(16)
   <223> n= G or A
<400> 5
   cagacatagg catggnttag tcctgttgcc a 31
<210> 6
   <211> 31
   <212> DNA
   <213> Homo sapiens
<220>
   <221> variation
   <222> (16)..(16)
   <223> n= T or C
<400> 6
   ctataagctc tgttanacac ttggccaagg t 31
<210> 7
   <211> 31
   <212> DNA
   <213> Homo sapiens
<220>
   <221> variation
   <222> (16)..(16)
   <223> n= G or A
<400> 7
   ttagaagagg cagacntaat ccagtgcatt t 31
<210> 8
   <211> 31
   <212> DNA
   <213> Homo sapiens
<220>
   <221> variation
   <222> (16)..(16)
   <223> n= C or T
<400> 8
   ttcacacaca aagacncata aagttgtacg t 31
<210> 9
   <211> 31
   <212> DNA
   <213> Homo sapiens
<220>
   <221> variation
   <222> (16)..(16)
   <223> n= C or T
<400> 9
   taaaatcaga gtagcnaatg agagctaaag t 31
<210> 10
   <211> 31
   <212> DNA
   <213> Homo sapiens
<220>
   <221> variation
   <222> (16)..(16)
   <223> n= C or G
<400> 10
   ccataatttt gactcngaat tacaaagtat t 31
<210> 11
   <211> 31
   <212> DNA
   <213> Homo sapiens
<220>
   <221> variation
   <222> (16)..(16)
   <223> n= C or T
<400> 11
   agcaacagtt tctaangggc aaataattta a 31
<210> 12
   <211> 31
   <212> DNA
   <213> Homo sapiens
<220>
   <221> variation
   <222> (16)..(16)
   <223> n= G or A
<400> 12
   ataaatggaa agaatnagaa caatgggaaa a 31
<210> 13
   <211> 31
   <212> DNA
   <213> Homo sapiens
<220>
   <221> variation
   <222> (16)..(16)
   <223> n= C or T
<400> 13
   cttgagtcat caggcnatag tcctaggact g 31
<210> 14
   <211> 31
   <212> DNA
   <213> Homo sapiens
<220>
   <221> variation
   <222> (16)..(16)
   <223> n= C or T
<400> 14
   tataagtttt ctgttnagaa aggccctgac a 31
<210> 15
   <211> 31
   <212> DNA
   <213> Homo sapiens
<220>
   <221> variation
   <222> (16)..(16)
   <223> n= A or G
<400> 15
   ttcatgtctt agtgantcca caattgattg t 31
<210> 16
   <211> 31
   <212> DNA
   <213> Homo sapiens
<220>
   <221> variation
   <222> (16)..(16)
   <223> n= G or T
<400> 16
   ggtgtttact tctaanatac aatgggagta t 31
<210> 17
   <211> 61
   <212> DNA
   <213> Homo sapiens
<220>
   <221> variation
   <222> (31)..(31)
   <223> n= A or G
<400> 17
   atgaaatccc taaggtcttc aagcaatctc ntgacaattt tttaaaattc acaaaagtac 60
   c 61
<210> 18
   <211> 61
   <212> DNA
   <213> Homo sapiens
<220>
   <221> variation
   <222> (31)..(31)
   <223> n= A or G
<400> 18
   agacagctgt ggaactggaa attaccaaac natttccaat tttaagagat ttggatcagg 60
   t 61
<210> 19
   <211> 31
   <212> DNA
   <213> Homo sapiens
<220>
   <221> variation
   <222> (16)..(16)
   <223> n= C or A
<400> 19
   catttctttg aaaatnaatg tggtttcacc a 31
<210> 20
   <211> 31
   <212> DNA
   <213> Homo sapiens
<220>
   <221> variation
   <222> (16)..(16)
   <223> n= A or T
<400> 20
   ctgattacag aagcantgcg tgatttttgt a 31
<210> 21
   <211> 31
   <212> DNA
   <213> Homo sapiens
<220>
   <221> variation
   <222> (16)..(16)
   <223> n= C or T
<400> 21
   agaatcagtg agatanggta tttctgtaca t 31
<210> 22
   <211> 31
   <212> DNA
   <213> Homo sapiens
<220>
   <221> variation
   <222> (16)..(16)
   <223> n= G or A
<400> 22
   ttggaagctg cgctcnattc tattcgctac a 31
<210> 23
   <211> 61
   <212> DNA
   <213> Homo sapiens
<220>
   <221> variation
   <222> (31)..(31)
   <223> n= A or G
<400> 23
   ctcgcagcag ttgtacttga tgtcgtgttt ntagcctggg gctttgatga tggcccactc 60
   g 61
<210> 24
   <211> 31
   <212> DNA
   <213> Homo sapiens
<220>
   <221> variation
   <222> (16)..(16)
   <223> n= A or G
<400> 24
   gctagtttgc ccccantggt gccctgctga g 31
<210> 25
   <211> 31
   <212> DNA
   <213> Homo sapiens
<220>
   <221> variation
   <222> (16)..(16)
   <223> n= C or T
<400> 25
   aataagtctt gtaagntata tcttctaaag c 31
<210> 26
   <211> 6367
   <212> DNA
   <213> Homo sapiens
<400> 26
   agacccgggg ctggctctgc tgctctcgcg atatttgcgc gagcctgctt ccttctttcc 60
   tcccttgcca gtccgcctgt cttcctcccc gtcttccctg cccggcctcc cccttcttcc 120
   cccgctggcc ccctccccgg agggataata tggtctccgg cgatggacgc cccaaaagca 180
   ggatacgcct ttgagtacct tattgaaaca ttaaatgaca gttcacataa gaagttcttc 240
   gatgtatcta aacttggcac caagtatgat gttctgcctt actcaatacg ggtcttgttg 300
   gaagctgctg tacgaaattg tgatggcttt ttaatgaaga aggaagatgt tatgaacatt 360
   ttagactgga aaaccaaaca aagcaatgtt gaagtgccct ttttccctgc ccgtgttctt 420
   cttcaagatt ttactggaat accagcaatg gtggattttg ctgctatgag ggaggcagtg     480
   aaaactcttg gaggtgatcc tgagaaagtc catcctgctt gtccgacaga tcttacagtt     540
   gaccattctt tacaaattga cttcagtaaa tgtgcaatac agaatgcacc aaatcctgga     600
   ggtggtgacc tgcagaaagc aggaaagctc tctccactta aagtgcagcc taagaagctt     660
   ccctgcagag gccagactac ctgccgagga tcttgtgatt ctggagaact aggccgaaac     720
   tcaggaacat tttcttcgca gattgagaat acacccatcc tgtgtccttt tcatttgcaa     780
   ccagtgcctg aacctgaaac agtgttaaaa aatcaagaag tagaattcgg cagaaatcga     840
   gagaggcttc agttttttaa gtggagttca agagttttta agaatgtggc agtgatccct     900
   cctggaactg gaatggctca tcaaataaac ttagaatatt tgtcaagagt ggtttttgaa     960
   gaaaaagacc tcctcttccc agacagtgta gtcggcacag attcacacat aacgatggtg    1020
   aatggtttag ggattctggg gtggggggtt ggaggcattg aaacagaagc agttatgctt    1080
   ggtctgccag tttctcttac tttaccagag gtggttggat gtgagttaac tgggtcatca    1140
   aacccttttg ttacatccat agatgttgtt cttggtatta caaagcacct caggcaagta    1200
   ggagtggctg gaaagtttgt tgagtttttt ggaagtggag tttcacaatt atctatagtt    1260
   gatcgaacta caatagcaaa catgtgtccg gaatatggtg ctatcctcag ctttttccct    1320
   gttgacaatg tgacattaaa acatttagaa catacaggtt ttagcaaagc caaactcgaa    1380
   tcaatggaaa cataccttaa agctgtgaaa ttgtttcgaa atgaccagaa ttcttcagga    1440
   gaacctgaat actcccaggt gatccagatt aatctgaatt caatagttcc atctgttagt    1500
   ggtccaaaaa gacctcagga tagagttgct gtgacagata tgaaaagcga tttccaggct    1560
   tgcttaaatg aaaaggttgg atttaaaggc ttccaaattg cagctgaaaa acaaaaggat    1620
   attgtctcca ttcattatga aggaagtgaa tataagctgt ctcatggatc agtggtcatt    1680
   gctgcagtta tcagttgtac caataattgc aatccatctg tcatgcttgc tgcaggtctt    1740
   ttggctaaaa aggctgttga agctggtctg cgtgttaaac cttatataag aacaagttta    1800
   tctccaggca gtgggatggt tacacattac ctcagttcaa gtggagtatt accatatcta    1860
   agtaagcttg gatttgaaat cgttggctat ggatgttcaa cttgtgtggg aaatacagca    1920
   cccttatcag acgcagtttt aaatgcagta aaacagggtg atttggttac ctgtggaatt    1980
   ttatctggaa acaaaaattt tgaaggtcgt ctttgtgatt gtgttcgtgc caattatctt    2040
   gcctctccac ccttagtggt agcttatgcc atagcaggca cagtgaatat agatttccag    2100
   acagaacctt taggtactga ccccaccggc aagaacattt acctgcatga tatttggcct    2160
   agtcgagaag aagttcatcg agtagaggaa gaacatgtta tactatccat gtttaaagca    2220
   ttaaaagata aaatagaaat ggggaataaa cggtggaatt ccttagaagc accggattca    2280
   gttttgtttc catgggactt aaagtctact tatatcagat gcccttcatt ttttgataaa    2340
   cttaccaaag agccaattgc actccaggct attgaaaatg cccatgtctt attatatttg    2400
   ggagactctg tcacaacaga tcatatatca cctgcaggaa gtatcgctag gaatagtgct    2460
   gccgctaagt atttgacaaa cagaggcctt acccctcgtg aattcaactc ttacggagct   2520
   cgaagaggta atgatgctgt aatgacaaga ggcacttttg caaatatcaa gctttttaat   2580
   aagtttattg gaaaaccagc tcctaaaaca attcattttc catcaggaca gacgctagat   2640
   gtatttgagg ctgcagagct gtaccagaaa gaaggtatcc cactgattat tttagcagga   2700
   aagaaatatg gttcaggaaa ctccagagac tgggctgcca aaggaccgta tttactgggt   2760
   gtgaaagctg ttttggccga aagttatgaa aaaatacaca aagatcattt gattggaatt   2820
   ggcatagctc cacttcagtt ccttccagga gaaaatgcag attccttggg cctctccggt   2880
   agagaaacat tttctttaac atttcctgaa gaactgtctc ctggaattac attgaatata   2940
   cagacaagca ctggaaaagt attcagcgtg attgcttcgt ttgaagatga tgtggaaata   3000
   acattataca aacatggagg attattaaac tttgtggcac gaaaattctc atagtatcta   3060
   cttaccatag atacctttca taactggtaa ctgcaaagcc ttttgtgctg gacccaggaa   3120
   tccttaccat ggagcagcag atagtcccag tatactcact tatctcatcc atggatgtaa   3180
   atgatgatga atcaacatag taactgaaat gaaatcttct tgattttaaa taatatacga   3240
   atggtgctat taatattgct aaaatcaacg tgtgaagtgt gttgtggaag agacctgtaa   3300
   gtatgggggg ggggcgatat tttatcagac cattttgtaa ataaaggcag aatttgtgtt   3360
   gaagattctt atatgaataa ccttcctgga tttgtttagt tttgcaccaa taaaactgtg   3420
   tactactgtt tgttggttta agagtagcag attgaaatat aagaagccag attagactct   3480
   aaaattgtgg ccattggaat ctcatttata aatggacctt ttaagtatat taattcctct   3540
   tcagaattga gctggacaca tttggcattc ttagtttgtc atataaccag gtttatcctt   3600
   agtctaactg caagggatag aacctgcccc aggtcacaat cattctgtcc aatccagcca   3660
   aggttccctc cacatatgaa gatggaccat ggcaggatac aactgattgt gtggcaccat   3720
   gtattagcag tgggaatatg tatcacatat gatgcagcct ttcatatttc agcagtttgc   3780
   cactgtgact gtctggcaag cccccagatg gcgttatatt aattggatta gattattttg   3840
   ctcaccttat gtaatactgt aacttcctat aacctaatat tttcggtatc attaaccaaa   3900
   atttcacact catagttgct aaagagaatg ttattcaatc attaaactct gaactgattt   3960
   cttctataca tttaaattat ccaccctcaa taatacgggt gctcaaccct atgcattttt   4020
   taagtgttgc tatttcttaa ttaaattgat ttcctgtcat tttgaatcat ttatcacctg   4080
   cgatgcatga ttctattaat tttgttatgt tactgtttta accaaagctg accgtaagga   4140
   taaaacactt aagttgttgc tgagtactat atatcctcaa tatgcatgtc tgcccatcac   4200
   atcaaatgtt ctgtcaacaa gatgtttggt aatttttttt aaaaaggttg gaaaattaat   4260
   tatagaaggt tctatactgt ttttttaatt aagaaactaa atctagcagg ctaaaggtta   4320
   attgtagtga ttttttttca catagatatc tttctatgac ctagttagtt actgcaattc   4380
   agaattagtt cacattgcat aaagaattac ttgttgtaag caaaatgctg aaactaccaa   4440
   accagtggat gaagaccact aagaactttg cacataatca tacaatcttt tgaaaatatt   4500
   ttgcaaatat gtgtttagac aataagatgg actagagttc gacaaaatga tttctttatt   4560
   taaattttgt ataagtattt tcttcgacac tttcaaatta tattgtgttc ttgatatatg    4620
   ctgtatattt atttgttagt gcatgtgttt ttaatttaca tgaaaacatg agttaggaga    4680
   aattacaggt tgaaagatga aatgcctgta tgtgctctga agaaatggta attccagatt    4740
   gtgcaggggg aaacaaatct attttgtttt gttttgtttt ttgagacgga gtcttgccct    4800
   gtggctgggc tggagtgcaa tggcgcgatc ttggctcact gcaacctccg cctcccgggt    4860
   tcaggggatt ctcctgcctc agcctcctga gtagctggga ctacaagcat gcaccaccac    4920
   gcccagctaa tttttgtagt tttagtagag atggggtttc accatgttgg ccagatgatc    4980
   tgaatctctt gacctcgtga tccgcccgcc ttggcctcca caagtgctgg gattacaggt    5040
   gtgagccacc acacccggcc tatattgttt tgaaagcata ctctatatat agttatgggc    5100
   agaggcacag gcatcctcag cagctgattc aggagatgat ggtaaagcta gctaactatg    5160
   aattaaacat tcacatatcc agtctacctg gtccagtaat aatacaagca aatcttgtat    5220
   ttcaggaaca aatcaaggtt ctcttaattt tttggcttat atacaatgaa gtaaaaactt    5280
   gataaacatg gtttcaaatt gaggaggaga gtcttggatg tatgttttaa tatgtatacc    5340
   ttataattct gcctctagcc aaatgctatg tttgcaaaat gtggcatctg ttagttttta    5400
   ttgtctgtgt cttctttgtt tactatacct tgggtaattt tgtgttacca aaaaaaaaaa    5460
   aaaaaaaagg aagtgtaatg tcagacacac aagaaaagca aatcagtgtt gtaagcttaa    5520
   agtacaattt caaaggtcac taccaacagc agggtttttt ttatactttg aaaacattat    5580
   gctacatatc attgccattt tcatattttg gggttttgct actcttatac aatggaatca     5640
   atggaaatgt catccagcca ctgaattgcc attattatat ctaaaaagtt tctaagatga    5700
   cagttatcac tattttgttt tatctccatg ctgacatttg aaagaaggta ctagtatccc    5760
   tctagccaga ttgcttagtt tttcgttggt aatcaaacaa cagttgtact aaaggaaagt    5820
   aaagctagga cctaaatcag aatcatagtt gcctgcatat atggtaacaa ggtcgtgtgc    5880
   atttgctttc acagtgatga gtgagaggat gagaagaaat tatttgacat ttttctgtgg    5940
   ttgaatagaa gacacctttc ttttgtcttt aggtttagga ggagatacta agatactgga    6000
   tgtttatcct atcttagttt ggttggagta ataagagaga agaagagggt ggactttggc    6060
   ttttcagtgt tttttcccct aaagagtgat attgctgacg tttctatcaa ttttacacat    6120
   aatatgtggc tatgaaacca tatatctcac ttaagtaaca aagtaatcac tttgtctatc    6180
   actaagtaat agacaaaaat cattgtctat tatttaaagc caacaaaaca gtgtaacagt    6240
   tttaagttca ataatgttaa gtattgtata gaaatatatt ggaggcaaag ttcagttgat    6300
   gacaattgtg tatatgttac tgatgctgta aattattttt aataaagaaa attgtattat    6360
   cacattt                                                              6367
<210> 27
   <211> 1235
   <212> DNA
   <213> Homo sapiens
<400> 27
   gcgggcgctg cggcccctgc tctacctcct agcgccggtg cgcggccgag gccgcactac      60
   ctgtctgcgg gaaagcggga tccaccccag gacgtcgggt cgctgccgac ataatgtcaa     120
   gtggaaacta tcagcagtca gaggctctta gcaaacccac tttcagtgag gaacaagcct     180
   ctgcgttagt ggagtcagtg tttgggttga aagtttccaa ggtccggcca cttcctagct     240
   atgatgacca aaactttcat gtctacgttt caaaaaccaa agatggccca actgaatatg     300
   tcctcaaaat aagcaacacc aaggctagca aaaatccaga cctgattgaa gtgcagaatc     360
   acatcatcat gtttctgaaa gccgctggat ttccaacagc ctctgtgtgt cacactaaag     420
   gagacaacac agcttctctc gtgtctgtag atagtggctc tgaaatcaaa agctacttgg     480
   tgaggctgct gacttacctc ccaggaagac ccatcgctga gcttcccgtc agcccccagc     540
   tattgtatga aattggaaaa ctagctgcca aattggataa gacactgcag agattccatc     600
   acccaaagtt aagtagtctt catcgggaga acttcatctg gaatctgaaa aatgttcctc     660
   ttctggagaa atacctgtat gccctgggcc agaatcgaaa ccgagagatt gttgagcatg     720
   tcattcatct gttcaaggag gaagtaatga ccaaattaag tcattttcga gaatgtatca     780
   atcacggaga tcttaatgac cataatattt taatagagtc cagcaagtca gcctctggaa     840
   atgctgaata tcaagtgtct gggattttag actttggtga catgagctat ggctactatg     900
   tgtttgaagt ggcaattacc atcatgtaca tgatgattga gagcaagagt cctatacaag     960
   taggaggcca tgtccttgca gggtttgaaa gcatcacccc actgacagct gtagagaagg    1020
   gtgctttgtt tttacttgta tgcagtcgtt tttgtcagtc acttgtcatg gctgcatact    1080
   cttgccagct atacccagag aacaaagact atctcatggt tactgcaaaa accgggtgga    1140
   aacacttaca gcaaatgttt gacatgggtc agaaagctgt agaagaaatc tggtttgaaa    1200
   ctgccaaatc ctatgaatct gggatctcca tgtga                               1235
<210> 28
   <211> 866
   <212> DNA
   <213> Homo sapiens
<400> 28
   gcgggcgctg cggcccctgc tctacctcct agcgccggtg cgcggccgag gccgcactac      60
   ctgtctgcgg gaaagcggga tccaccccag gacgtcgggt cgctgccgac ataatgtcaa     120
   gtggaaacta tcagcagtca gaggctctta gcaaacccac tttcagtgag gaacaagcct     180
   ctgcgttagt ggagtcagtg tttgggttga aagtttccaa ggtccggcca cttcctagct     240
   atgatgacca aaactttcat gtctacgttt caaaaaccaa agatggccca actgaatatg     300
   tcctcaaaat aagcaacacc aaggctagca aaaatccaga cctgattgaa gtgcagaatc     360
   acatcatcat gtttctgaaa gccgctggat ttccaacagc ctctgtgtgt cacactaaag     420
   gagacaacac agcttctctc gtgtctgtag atagtggctc tgaaatcaaa agctacttgg     480
   tgaggctgct gacttacctc ccaggaagac ccatcgctga gcttcccgtc agcccccagc     540
   tattgtatga aattggaaaa ctagctgcca aattggataa gacactgcag agattccatc     600
   acccaaagtt aagtagtctt catcgggaga acttcatctg gaatctgaaa aatgttcctc     660
   ttctggagaa atacctgtat gccctgggcc agaatcgaaa ccgagagatt gttgagcatg     720
   tcattcatct gttcaaggag gaagtaatga ccaaattaag tcattttcga gaatgaccta     780
   gcaccgtgcc aggcccctag aagacccagt aaagatctgt tgaataaact gtaagaatga     840
   acacaccaaa aaaaaaaaaa aaaaaa                                          866
<210> 29
   <211> 1189
   <212> DNA
   <213> Homo sapiens
<400> 29
   gcgcatgcgc gggggccata ttagcagcgg ttattcggtg agcggtggtg gtttattctt      60
   ccgtggagtt aagggctccg tggacatctc aggtcttcag ggtcttccat ctggaactat     120
   ataaagttca gaaaacatgt ctcgaagata tgactccagg accactatat tttctccaga     180
   aggtcgctta taccaagttg aatatgccat ggaagctatt ggacatgcag gcacctgttt     240
   gggaatttta gcaaatgatg gtgttttgct tgcagcagag agacgcaaca tccacaagct     300
   tcttgatgaa gtcttttttt ctgaaaaaat ttataaactc aatgaggaca tggcttgcag     360
   tgtggcaggc ataacttctg atgctaatgt tctgactaat gaactaaggc tcattgctca     420
   aaggtattta ttacagtatc aggagccaat accttgtgag cagttggtta cagcgctgtg     480
   tgatatcaaa caagcttata cacaatttgg aggaaaacgt ccctttggtg tttcattgct     540
   gtacattggc tgggataagc actatggctt tcagctctat cagagtgacc ctagtggaaa     600
   ttacggggga tggaaggcca catgcattgg aaataatagc gctgcagctg tgtcaatgtt     660
   gaaacaagac tataaagaag gagaaatgac cttgaagtca gcacttgctt tagctatcaa     720
   agtactaaat aagaccatgg atgttagtaa actctctgct gaaaaagtgg aaattgcaac     780
   actaacaaga gagaatggaa agacagtaat cagagttctc aaacaaaaag aagtggagca     840
   gttgatcaaa aaacatgagg aagaagaagc caaagctgag cgtgagaaga aagaaaaaga     900
   acagaaagaa aaggataaat agaatcagag attttattac tcatttgggg caccatttca     960
   gtgtaaaagc agtcctactc ttccacacta ggaaggcttt acttttttta actggtgcag    1020
   tgggaaaata ggacattaca tactgaattg ggtccttgtc atttctgtcc aattgaatac    1080
   tttattgtaa cgatgatggt tacccttcat ggacgtctta atcttccaca cacatcccct    1140
   ttttttggaa taaaatttgg aaaatggaaa tgaaaaaaaa aaaaaaaaa               1189
<210> 30
   <211> 2468
   <212> DNA
   <213> Homo sapiens
<400> 30
   ccacgcgtcc gggcgggagc tgtggcgcgg agcggcccct ctgctgcgtc tgccctcgtt      60
   ttgtctcacg actcacactc agtgctccat tccccaagag ttcgcgttcc ccgcgcggcg     120
   gtcgagaggc ggctgcccgc ggtcccgcgc gggcgcgggg cgatggcggc gcgggggtca    180
   gggccccgcg cgctccgcct gctgctcttg gtccagctgg tcgcggggcg ctgcggtcta    240
   gcgggcgcgg cgggcggcgc gcagagagga ttatctgaac cttcttctat tgcaaaacat    300
   gaagatagtt tgcttaagga tttatttcaa gactacgaaa gatgggttcg tcctgtggaa    360
   cacctgaatg acaaaataaa aataaaattt ggacttgcaa tatctcaatt ggtggatgtg    420
   gatgagaaaa atcagttaat gacaacaaac gtctggttga aacaggaatg gatagatgta    480
   aaattaagat ggaaccctga tgactatggt ggaataaaag ttatacgtgt tccttcagac    540
   tctgtctgga caccagacat cgttttgttt gataatgcag atggacgttt tgaagggacc    600
   agtacgaaaa cagtcatcag gtacaatggc actgtcacct ggactccacc ggcaaactac    660
   aaaagttcct gtaccataga tgtcacgttt ttcccatttg accttcagaa ctgttccatg    720
   aaatttggtt cttggactta tgatggatca caggttgata taattctaga ggaccaagat    780
   gtagacaaga gagatttttt tgataatgga gaatgggaga ttgtgagtgc aacagggagc    840
   aaaggaaaca gaaccgacag ctgttgctgg tatccgtatg tcacttactc atttgtaatc    900
   aagcgcctgc ctctctttta taccttgttc cttataatac cctgtattgg gctctcattt    960
   ttaactgtac ttgtcttcta tcttccttca aatgaaggtg aaaagatttg tctctgcact   1020
   tcagtacttg tgtctttgac tgtcttcctt ctggttattg aagagatcat accatcatct   1080
   tcaaaagtca tacctctaat tggagagtat ctggtattta ccatgatttt tgtgacactg   1140
   tcaattatgg taaccgtctt cgctatcaac attcatcatc gttcttcctc aacacataat   1200
   gccatggcgc ctttggtccg caagatattt cttcacacgc ttcccaaact gctttgcatg   1260
   agaagtcatg tagacaggta cttcactcag aaagaggaaa ctgagagtgg tagtggacca   1320
   aaatcttcta gaaacacatt ggaagctgcg ctcgattcta ttcgctacat tacaagacac   1380
   atcatgaagg aaaatgatgt ccgtgaggtt gttgaagatt ggaaattcat agcccaggtt   1440
   cttgatcgga tgtttctgtg gacttttctt ttcgtttcaa ttgttggatc tcttgggctt   1500
   tttgttcctg ttatttataa atgggcaaat atattaatac cagttcatat tggaaatgca   1560
   aataagtgaa gcctcccaag ggactgaagt atacatttag ttaacacaca tatatctgat   1620
   ggcacctata aaattatgaa aatgtaagtt atgtgttaaa tttagtgcaa gctttaacag   1680
   actaagttgc taacctcaat ttatgttaac agatgatcca tttgaacagt tggctgtatg   1740
   actgaagtaa taactgatga gatacatttg atcttgtaaa aatagcaaaa tattatctga   1800
   actggactag tgaaaaatct agtatttgta tcctggcaaa taatactaat ttataatcca   1860
   cagtaaagtt catcctttga ctgtgctgga gaattccagt tgtatttgaa gactgatttt   1920
   aaaacttttc tgcatttggt aaaggtatgt aaactttcct gtactcactg agtaacagct   1980
   aatctttaat ataatattat actgctatat ttaaaaagct gactacttga tataattact   2040
   taatgtgatg cttgatataa taattactta atgtggccgg gcacggtggc tcacacctgt   2100
   aatcccagca ctttgggagg tcgaggtggg cgtatcacct gaggttggga gttcgagacc   2160
   agcctgacca acgtggagaa accccgtctc tactaaaaat atgaaattag ccagggtggt   2220
   ggtgcacacc tgtaatccca gctacctggg aggctgcggc aggagaatcg cttgaaccca 2280
   ggtggcggag gttgcggtga gctgagatca cgccattgca ctccagcctg ggcaacaaga 2340
   gcaaaactca gtctcaaata ataataataa caacaactta atgtgctgct gcttttccat 2400
   aaccaacatt ttaaaaataa atgaaaaaca ggaaaaaaaa aaaaaaaaaa aaaaaaaaaa 2460
   aaaaaaaa 2468
<210> 31
   <211> 3020
   <212> DNA
   <213> Homo sapiens
<400> 31
   gtcctcccgc gggtccgagg gcgctggaaa cccagcggcg gcgaagcgga gaggagcccc    60
   gcgcgtctcc gcccgcacgg ctccaggtct ggggtctgcg ctggagccgc gcggggagag   120
   gccgtctctg cgaccgccgc gcccgctccc gaccgtccgg gtccgcggcc agcccggcca   180
   ccagccatgg gctctggccc gctctcgctg cccctggcgc tgtcgccgcc gcggctgctg   240
   ctgctgctgc tgctgtctct gctgccagtg gccagggcct cagaggctga gcaccgtcta   300
   tttgagcggc tgtttgaaga ttacaatgag atcatccggc ctgtagccaa cgtgtctgac   360
   ccagtcatca tccatttcga ggtgtccatg tctcagctgg tgaaggtgga tgaagtaaac   420
   cagatcatgg agaccaacct gtggctcaag caaatctgga atgactacaa gctgaagtgg   480
   aacccctctg actatggtgg ggcagagttc atgcgtgtcc ctgcacagaa gatctggaag   540
   ccagacattg tgctgtataa caatgctgtt ggggatttcc aggtggacga caagaccaaa   600
   gccttactca agtacactgg ggaggtgact tggatacctc cggccatctt taagagctcc   660
   tgtaaaatcg acgtgaccta cttcccgttt gattaccaaa actgtaccat gaagttcggt   720
   tcctggtcct acgataaggc gaaaatcgat ctggtcctga tcggctcttc catgaacctc   780
   aaggactatt gggagagcgg cgagtgggcc atcatcaaag ccccaggcta caaacacgac   840
   atcaagtaca actgctgcga ggagatctac cccgacatca catactcgct gtacatccgg   900
   cgcctgccct tgttctacac catcaacctc atcatcccct gcctgctcat ctccttcctc   960
   actgtgctcg tcttctacct gccctccgac tgcggtgaga aggtgaccct gtgcatttct  1020
   gtcctcctct ccctgacggt gtttctcctg gtgatcactg agaccatccc ttccacctcg  1080
   ctggtcatcc ccctgattgg agagtacctc ctgttcacca tgatttttgt aaccttgtcc  1140
   atcgtcatca ccgtcttcgt gctcaacgtg cactacagaa ccccgacgac acacacaatg  1200
   ccctcatggg tgaagactgt attcttgaac ctgctcccca gggtcatgtt catgaccagg  1260
   ccaacaagca acgagggcaa cgctcagaag ccgaggcccc tctacggtgc cgagctctca  1320
   aatctgaatt gcttcagccg cgcagagtcc aaaggctgca aggagggcta cccctgccag  1380
   gacgggatgt gtggttactg ccaccaccgc aggataaaaa tctccaattt cagtgctaac  1440
   ctcacgagaa gctctagttc tgaatctgtt gatgctgtgc tgtccctctc tgctttgtca  1500
   ccagaaatca aagaagccat ccaaagtgtc aagtatattg ctgaaaatat gaaagcacaa  1560
   aatgaagcca aagagattca agatgattgg aagtatgttg ccatggtgat tgatcgtatt 1620
   tttctgtggg ttttcaccct ggtgtgcatt ctagggacag caggattgtt tctgcaaccc 1680
   ctgatggcca gggaagatgc ataagcacta agctgtgtgc ctgcctggga gacttccttg 1740
   tgtcagggca ggaggaggct gcttcctagt aagaacgtac tttctgttat caagctacca 1800
   gctttgtttt tggcatttcg aggtttactt attttccact tatcttggaa tcatgcaaaa 1860
   aaaaaaatgt caagagtatt tattaccgat aaatgaacat ttaactagcc tttttggtat 1920
   ggtaaagaga tgtcaaaatg tgattctatg tgattagtat gctatgctat ggaatataca 1980
   tgtaaaaatg tttcctttta gttgttgaaa caaaactgga tagaaaaatg ctgttcagaa 2040
   atatgaaaag tcattcagtt atcactacag atctcccagt aatttttctt atttagccca 2100
   taatctcttt gaaggtttat actaattcag caatccccca tcgttaccca tttcttacca 2160
   tgcatttctc gttctttact gggtctaaag ggctatgcct ccatttcaga gagcttcaac 2220
   tacttctctt gcatacttct aaattatact atgagaaatc atgcctagtt attcattgtt 2280
   aatataactg tcttagtaca ccataaactg ggtggattat aaacaacaga aacttctcag 2340
   ttttggaggt tgggaggtcc aaggtcaagg caccagcaaa tttggtgtct ggtgagggtc 2400
   ctcttcctca aagggtgcct tctagctgtg tcctcacatg actgaaggga ctagctatct 2460
   ctgtggggtc tattttataa gggcactaac cccattcatg agagcagagc ccccatggcc 2520
   taatcacctt tccaaggccc caccttctat ctaagacaat cacgctggga ataggtttca 2580
   acatatgaat tgggggagga cacatttgga ccacagcatg aacctttaga acagggtttc 2640
   tcagccttag cactacggac attttgggct ggataaatat gtgttggtac agaatggggg 2700
   tatcctgtgc attgtaggat ctttagcagt accctagcct caactcacta gatgccaatg 2760
   acataccttg cttcttcacc agttatgata accaagaatg tctccattgt taaatgtccc 2820
   cttaggagca aaattgcccc tggttgagaa acattgcttt agacaaattg ttaagagtat 2880
   catgtactac acttctgaaa cttaacgtga tcatcaccac tgacagatga ttcacagaga 2940
   gagactgttt gaatcttgtc tcactagttt ttcctgtgca aaaataaaat ggacagaatt 3000
   gcaaaaaaaa aaaaaaaaaa 3020
<210> 32
   <211> 2447
   <212> DNA
   <213> Homo sapiens
<400> 32
   ccgccagcaa acctcggggg ccaggaccgg cgctcactcg accgcgcggc tcacgggtgc 60
   cctgtgaccc cacagcggac gtcgcggcgg ctgccacccg gccccgccgg ccatgaggcg 120
   cgcgccttcc ctggtccttt tcttcctggt cgccctttgc gggcgcggga actgccgcgt 180
   ggccaatgcg gaggaaaagc tgatggacga ccttctgaac aaaacccgtt acaataacct 240
   gatccgccca gccaccagct cctcacagct catctccatc aagctgcagc tctccctggc 300
   ccagcttatc agcgtgaatg agcgagagca gatcatgacc accaatgtct ggctgaaaca 360
   ggaatggact gattaccgcc tgacctggaa cagctcccgc tacgagggtg tgaacatcct 420
   gaggatccct gcaaagcgca tctggttgcc tgacatcgtg ctttacaaca acgccgacgg 480
   gacctatgag gtgtctgtct acaccaactt gatagtccgg tccaacggca gcgtcctgtg 540
   gctgccccct gccatctaca agagcgcctg caagattgag gtgaagtact ttcccttcga 600
   ccagcagaac tgcaccctca agttccgctc ctggacctat gaccacacgg agatagacat 660
   ggtcctcatg acgcccacag ccagcatgga tgactttact cccagtggtg agtgggacat 720
   agtggccctc ccagggagaa ggacagtgaa cccacaagac cccagctacg tggacgtgac 780
   ttacgacttc atcatcaagc gcaagcctct gttctacacc atcaacctca tcatcccctg 840
   cgtgctcacc accttgctgg ccatcctcgt cttctacctg ccatccgact gcggcgagaa 900
   gatgacactg tgcatctcag tgctgctggc actgacattc ttcctgctgc tcatctccaa 960
   gatcgtgcca cccacctccc tcgatgtgcc tctcatcggc aagtacctca tgttcaccat 1020
   ggtgctggtc accttctcca tcgtcaccag cgtctgtgtg ctcaatgtgc accaccgctc 1080
   gcccagcacc cacaccatgg caccctgggt caagcgctgc ttcctgcaca agctgcctac 1140
   cttcctcttc atgaagcgcc ctggccccga cagcagcccg gccagagcct tcccgcccag 1200
   caagtcatgc gtgaccaagc ccgaggccac cgccacctcc accagcccct ccaacttcta 1260
   tgggaactcc atgtactttg tgaaccccgc ctctgcagct tccaagtctc cagccggctc 1320
   taccccggtg gctatcccca gggatttctg gctgcggtcc tctgggaggt tccgacagga 1380
   tgtgcaggag gcattagaag gtgtcagctt catcgcccag cacatgaaga atgacgatga 1440
   agaccagagt gtcgttgagg actggaagta cgtggctatg gtggtggacc ggctgttcct 1500
   gtgggtgttc atgtttgtgt gcgtcctggg cactgtgggg ctcttcctac cgcccctctt 1560
   ccagacccat gcagcttctg aggggcccta cgctgcccag cgtgactgag ggccccctgg 1620
   gttgtggggt gagaggatgt gagtggccgg gtgggcactt tgctgcttct ttctgggttg 1680
   tggccgatga ggcctaagta aatatgtgag cattggccat caaccccatc aaaccagccc 1740
   aagccgtgga acaggcaagg atgggggcct gggctgtcct ctctgaatgc cttggaggga 1800
   tcccaggaag ccccagtagg agggagcttc agacagttca attctggcct gtcttccttc 1860
   cctgcaccgg gcaatgggga taaagatgac ttcgtagcag cacctactat gcttcaggca 1920
   tggtgccggc ctgcctctcc atcaccatct ctctccactt ccccttgtcc agttcctcac 1980
   acacttctag attcttccca gctcagaggc ttggcatttg ccatatcatc atcttttttt 2040
   ttttctttta aacggagtct tgctatatcg cccaggctca agtgattctc ctgcctgaac 2100
   ctcccaagta gctgggatta cagaaagccg ccaccgtgcc cagctaattt ttgtattttt 2160
   gttggccagg ctgatttcga actcctgacc tcagatgacc cacctgcctc ggcctcccaa 2220
   agtgctggga ttacaggtgt gagccactat gcctggcctt ccctatcttc tacctgaacc 2280
   ttcttcctct tcccccaggg cttcccaagc tctttccaca gccacatcag tcagggcatg 2340
   gctccaatcc attctttgct ccaatgtcac ctcctctgag aggccttccc taaccaccca 2400
   atcattctaa agcagcctcc ctacagtcac gttaccctgc ctccttc 2447
<210> 33
   <211> 963
   <212> PRT
   <213> Homo sapiens
<400> 33
   Met Asp Ala Pro Lys Ala Gly Tyr Ala Phe Glu Tyr Leu Ile Glu Thr
   1               5                   10                  15
   Leu Asn Asp Ser Ser His Lys Lys Phe Phe Asp Val Ser Lys Leu Gly
               20                  25                  30
   Thr Lys Tyr Asp Val Leu Pro Tyr Ser Ile Arg Val Leu Leu Glu Ala
           35                  40                  45
   Ala Val Arg Asn Cys Asp Gly Phe Leu Met Lys Lys Glu Asp Val Met
       50                  55                  60
   Asn Ile Leu Asp Trp Lys Thr Lys Gln Ser Asn Val Glu Val Pro Phe
   65                  70                  75                  80
   Phe Pro Ala Arg Val Leu Leu Gln Asp Phe Thr Gly Ile Pro Ala Met
                   85                  90                  95
   Val Asp Phe Ala Ala Met Arg Glu Ala Val Lys Thr Leu Gly Gly Asp
               100                 105                 110
   Pro Glu Lys Val His Pro Ala Cys Pro Thr Asp Leu Thr Val Asp His
           115                 120                 125
   Ser Leu Gln Ile Asp Phe Ser Lys Cys Ala Ile Gln Asn Ala Pro Asn
       130                 135                 140
   Pro Gly Gly Gly Asp Leu Gln Lys Ala Gly Lys Leu Ser Pro Leu Lys
   145                 150                 155                 160
   Val Gln Pro Lys Lys Leu Pro Cys Arg Gly Gln Thr Thr Cys Arg Gly
                   165                 170                 175
   Ser Cys Asp Ser Gly Glu Leu Gly Arg Asn Ser Gly Thr Phe Ser Ser
               180                 185                 190
   Gln Ile Glu Asn Thr Pro Ile Leu Cys Pro Phe His Leu Gln Pro Val
           195                 200                 205
   Pro Glu Pro Glu Thr Val Leu Lys Asn Gln Glu Val Glu Phe Gly Arg
       210                 215                 220
   Asn Arg Glu Arg Leu Gln Phe Phe Lys Trp Ser Ser Arg Val Phe Lys
   225                 230                 235                 240
   Asn Val Ala Val Ile Pro Pro Gly Thr Gly Met Ala His Gln Ile Asn
                   245                 250                 255
   Leu Glu Tyr Leu Ser Arg Val Val Phe Glu Glu Lys Asp Leu Leu Phe
               260                 265                 270
   Pro Asp Ser Val Val Gly Thr Asp Ser His Ile Thr Met Val Asn Gly
           275                 280                 285
   Leu Gly Ile Leu Gly Trp Gly Val Gly Gly Ile Glu Thr Glu Ala Val
       290                 295                 300
   Met Leu Gly Leu Pro Val Ser Leu Thr Leu Pro Glu Val Val Gly Cys
   305                 310                 315                 320
   Glu Leu Thr Gly Ser Ser Asn Pro Phe Val Thr Ser Ile Asp Val Val
                   325                 330                 335
   Leu Gly Ile Thr Lys His Leu Arg Gln Val Gly Val Ala Gly Lys Phe
               340                 345                 350
   Val Glu Phe Phe Gly Ser Gly Val Ser Gln Leu Ser Ile Val Asp Arg
           355                 360                 365
   Thr Thr Ile Ala Asn Met Cys Pro Glu Tyr Gly Ala Ile Leu Ser Phe
       370                 375                 380
   Phe Pro Val Asp Asn Val Thr Leu Lys His Leu Glu His Thr Gly Phe
   385                 390                 395                 400
   Ser Lys Ala Lys Leu Glu Ser Met Glu Thr Tyr Leu Lys Ala Val Lys
                   405                 410                 415
   Leu Phe Arg Asn Asp Gln Asn Ser Ser Gly Glu Pro Glu Tyr Ser Gln
               420                 425                 430
   Val Ile Gln Ile Asn Leu Asn Ser Ile Val Pro Ser Val Ser Gly Pro
           435                 440                 445
   Lys Arg Pro Gln Asp Arg Val Ala Val Thr Asp Met Lys Ser Asp Phe
       450                 455                 460
   Gln Ala Cys Leu Asn Glu Lys Val Gly Phe Lys Gly Phe Gln Ile Ala
   465                 470                 475                 480
   Ala Glu Lys Gln Lys Asp Ile Val Ser Ile His Tyr Glu Gly Ser Glu
                   485                 490                 495
   Tyr Lys Leu Ser His Gly Ser Val Val Ile Ala Ala Val Ile Ser Cys
               500                 505                 510
   Thr Asn Asn Cys Asn Pro Ser Val Met Leu Ala Ala Gly Leu Leu Ala
           515                 520                 525
   Lys Lys Ala Val Glu Ala Gly Leu Arg Val Lys Pro Tyr Ile Arg Thr
       530                 535                 540
   Ser Leu Ser Pro Gly Ser Gly Met Val Thr His Tyr Leu Ser Ser Ser
   545                 550                 555                 560
   Gly Val Leu Pro Tyr Leu Ser Lys Leu Gly Phe Glu Ile Val Gly Tyr
                   565                 570                 575
   Gly Cys Ser Thr Cys Val Gly Asn Thr Ala Pro Leu Ser Asp Ala Val
               580                 585                 590
   Leu Asn Ala Val Lys Gln Gly Asp Leu Val Thr Cys Gly Ile Leu Ser
           595                 600                 605
   Gly Asn Lys Asn Phe Glu Gly Arg Leu Cys Asp Cys Val Arg Ala Asn
       610                 615                 620
   Tyr Leu Ala Ser Pro Pro Leu Val Val Ala Tyr Ala Ile Ala Gly Thr
   625                 630                 635                 640
   Val Asn Ile Asp Phe Gln Thr Glu Pro Leu Gly Thr Asp Pro Thr Gly
                   645                 650                 655
   Lys Asn Ile Tyr Leu His Asp Ile Trp Pro Ser Arg Glu Glu Val His
               660                 665                 670
   Arg Val Glu Glu Glu His Val Ile Leu Ser Met Phe Lys Ala Leu Lys
           675                 680                 685
   Asp Lys Ile Glu Met Gly Asn Lys Arg Trp Asn Ser Leu Glu Ala Pro
       690                 695                 700
   Asp Ser Val Leu Phe Pro Trp Asp Leu Lys Ser Thr Tyr Ile Arg Cys
   705                 710                 715                 720
   Pro Ser Phe Phe Asp Lys Leu Thr Lys Glu Pro Ile Ala Leu Gln Ala
                   725                 730                 735
   Ile Glu Asn Ala His Val Leu Leu Tyr Leu Gly Asp Ser Val Thr Thr
               740                 745                 750
   Asp His Ile Ser Pro Ala Gly Ser Ile Ala Arg Asn Ser Ala Ala Ala
           755                 760                 765
   Lys Tyr Leu Thr Asn Arg Gly Leu Thr Pro Arg Glu Phe Asn Ser Tyr
       770                 775                 780
   Gly Ala Arg Arg Gly Asn Asp Ala Val Met Thr Arg Gly Thr Phe Ala
   785 790 795 800
   Asn Ile Lys Leu Phe Asn Lys Phe Ile Gly Lys Pro Ala Pro Lys Thr
   805 810 815
   Ile His Phe Pro Ser Gly Gln Thr Leu Asp Val Phe Glu Ala Ala Glu
   820 825 830
   Leu Tyr Gln Lys Glu Gly Ile Pro Leu Ile Ile Leu Ala Gly Lys Lys
   835 840 845
   Tyr Gly Ser Gly Asn Ser Arg Asp Trp Ala Ala Lys Gly Pro Tyr Leu
   850 855 860
   Leu Gly Val Lys Ala Val Leu Ala Glu Ser Tyr Glu Lys Ile His Lys
   865 870 875 880
   Asp His Leu Ile Gly Ile Gly Ile Ala Pro Leu Gln Phe Leu Pro Gly
   885 890 895
   Glu Asn Ala Asp Ser Leu Gly Leu Ser Gly Arg Glu Thr Phe Ser Leu
   900 905 910
   Thr Phe Pro Glu Glu Leu Ser Pro Gly Ile Thr Leu Asn Ile Gln Thr
   915 920 925
   Ser Thr Gly Lys Val Phe Ser Val Ile Ala Ser Phe Glu Asp Asp Val
   930 935 940
   Glu Ile Thr Leu Tyr Lys His Gly Gly Leu Leu Asn Phe Val Ala Arg
   945 950 955 960
   Lys Phe Ser
<210> 34
   <211> 373
   <212> PRT
   <213> Homo sapiens
<400> 34
   Met Ser Ser Gly Asn Tyr Gln Gln Ser Glu Ala Leu Ser Lys Pro Thr
   1 5 10 15
   Phe Ser Glu Glu Gln Ala Ser Ala Leu Val Glu Ser Val Phe Gly Leu
   20 25 30
   Lys Val Ser Lys Val Arg Pro Leu Pro Ser Tyr Asp Asp Gln Asn Phe
   35 40 45
   His Val Tyr Val Ser Lys Thr Lys Asp Gly Pro Thr Glu Tyr Val Leu
   50 55 60
   Lys Ile Ser Asn Thr Lys Ala Ser Lys Asn Pro Asp Leu Ile Glu Val
   65                  70                  75                  80
   Gln Asn His Ile Ile Met Phe Leu Lys Ala Ala Gly Phe Pro Thr Ala
                   85                  90                  95
   Ser Val Cys His Thr Lys Gly Asp Asn Thr Ala Ser Leu Val Ser Val
               100                 105                 110
   Asp Ser Gly Ser Glu Ile Lys Ser Tyr Leu Val Arg Leu Leu Thr Tyr
           115                 120                 125
   Leu Pro Gly Arg Pro Ile Ala Glu Leu Pro Val Ser Pro Gln Leu Leu
       130                 135                 140
   Tyr Glu Ile Gly Lys Leu Ala Ala Lys Leu Asp Lys Thr Leu Gln Arg
   145                 150                 155                 160
   Phe His His Pro Lys Leu Ser Ser Leu His Arg Glu Asn Phe Ile Trp
                   165                 170                 175
   Asn Leu Lys Asn Val Pro Leu Leu Glu Lys Tyr Leu Tyr Ala Leu Gly
               180                 185                 190
   Gln Asn Arg Asn Arg Glu Ile Val Glu His Val Ile His Leu Phe Lys
           195                 200                 205
   Glu Glu Val Met Thr Lys Leu Ser His Phe Arg Glu Cys Ile Asn His
       210                 215                 220
   Gly Asp Leu Asn Asp His Asn Ile Leu Ile Glu Ser Ser Lys Ser Ala
   225                 230                 235                 240
   Ser Gly Asn Ala Glu Tyr Gln Val Ser Gly Ile Leu Asp Phe Gly Asp
                   245                 250                 255
   Met Ser Tyr Gly Tyr Tyr Val Phe Glu Val Ala Ile Thr Ile Met Tyr
               260                 265                 270
   Met Met Ile Glu Ser Lys Ser Pro Ile Gln Val Gly Gly His Val Leu
           275                 280                 285
   Ala Gly Phe Glu Ser Ile Thr Pro Leu Thr Ala Val Glu Lys Gly Ala
       290                 295                 300
   Leu Phe Leu Leu Val Cys Ser Arg Phe Cys Gln Ser Leu Val Met Ala
   305                 310                 315                 320
   Ala Tyr Ser Cys Gln Leu Tyr Pro Glu Asn Lys Asp Tyr Leu Met Val
                   325                 330                 335
   Thr Ala Lys Thr Gly Trp Lys His Leu Gln Gln Met Phe Asp Met Gly
               340                 345                 350
   Gln Lys Ala Val Glu Glu Ile Trp Phe Glu Thr Ala Lys Ser Tyr Glu
           355                 360                 365
   Ser Gly Ile Ser Met
       370
<210> 35
   <211> 220
   <212> PRT
   <213> Homo sapiens
<400> 35
   Met Ser Ser Gly Asn Tyr Gln Gln Ser Glu Ala Leu Ser Lys Pro Thr
   1               5                   10                  15
   Phe Ser Glu Glu Gln Ala Ser Ala Leu Val Glu Ser Val Phe Gly Leu
               20                  25                  30
   Lys Val Ser Lys Val Arg Pro Leu Pro Ser Tyr Asp Asp Gln Asn Phe
           35                  40                  45
   His Val Tyr Val Ser Lys Thr Lys Asp Gly Pro Thr Glu Tyr Val Leu
       50                  55                  60
   Lys Ile Ser Asn Thr Lys Ala Ser Lys Asn Pro Asp Leu Ile Glu Val
   65                  70                  75                  80
   Gln Asn His Ile Ile Met Phe Leu Lys Ala Ala Gly Phe Pro Thr Ala
                   85                  90                  95
   Ser Val Cys His Thr Lys Gly Asp Asn Thr Ala Ser Leu Val Ser Val
               100                 105                 110
   Asp Ser Gly Ser Glu Ile Lys Ser Tyr Leu Val Arg Leu Leu Thr Tyr
           115                 120                 125
   Leu Pro Gly Arg Pro Ile Ala Glu Leu Pro Val Ser Pro Gln Leu Leu
       130                 135                 140
   Tyr Glu Ile Gly Lys Leu Ala Ala Lys Leu Asp Lys Thr Leu Gln Arg
   145                 150                 155                 160
   Phe His His Pro Lys Leu Ser Ser Leu His Arg Glu Asn Phe Ile Trp
                   165                 170                 175
   Asn Leu Lys Asn Val Pro Leu Leu Glu Lys Tyr Leu Tyr Ala Leu Gly
               180                 185                 190
   Gln Asn Arg Asn Arg Glu Ile Val Glu His Val Ile His Leu Phe Lys
           195                 200                 205
   Glu Glu Val Met Thr Lys Leu Ser His Phe Arg Glu
       210                 215                 220
<210> 36
   <211> 261
   <212> PRT
   <213> Homo sapiens
<400> 36
   Met Ser Arg Arg Tyr Asp Ser Arg Thr Thr Ile Phe Ser Pro Glu Gly
   1               5                   10                  15
   Arg Leu Tyr Gln Val Glu Tyr Ala Met Glu Ala Ile Gly His Ala Gly
               20                  25                  30
   Thr Cys Leu Gly Ile Leu Ala Asn Asp Gly Val Leu Leu Ala Ala Glu
           35                  40                  45
   Arg Arg Asn Ile His Lys Leu Leu Asp Glu Val Phe Phe Ser Glu Lys
       50                  55                  60
   Ile Tyr Lys Leu Asn Glu Asp Met Ala Cys Ser Val Ala Gly Ile Thr
   65                  70                  75                  80
   Ser Asp Ala Asn Val Leu Thr Asn Glu Leu Arg Leu Ile Ala Gln Arg
                   85                  90                  95
   Tyr Leu Leu Gln Tyr Gln Glu Pro Ile Pro Cys Glu Gln Leu Val Thr
               100                 105                 110
   Ala Leu Cys Asp Ile Lys Gln Ala Tyr Thr Gln Phe Gly Gly Lys Arg
           115                 120                 125
   Pro Phe Gly Val Ser Leu Leu Tyr Ile Gly Trp Asp Lys His Tyr Gly
       130                 135                 140
   Phe Gln Leu Tyr Gln Ser Asp Pro Ser Gly Asn Tyr Gly Gly Trp Lys
   145                 150                 155                 160
   Ala Thr Cys Ile Gly Asn Asn Ser Ala Ala Ala Val Ser Met Leu Lys
                   165                 170                 175
   Gln Asp Tyr Lys Glu Gly Glu Met Thr Leu Lys Ser Ala Leu Ala Leu
               180                 185                 190
   Ala Ile Lys Val Leu Asn Lys Thr Met Asp Val Ser Lys Leu Ser Ala
           195                 200                 205
   Glu Lys Val Glu Ile Ala Thr Leu Thr Arg Glu Asn Gly Lys Thr Val
       210                 215                 220
   Ile Arg Val Leu Lys Gln Lys Glu Val Glu Gln Leu Ile Lys Lys His
   225                 230                 235                 240
   Glu Glu Glu Glu Ala Lys Ala Glu Arg Glu Lys Lys Glu Lys Glu Gln
                   245                 250                 255
   Lys Glu Lys Asp Lys
               260
<210> 37
   <211> 468
   <212> PRT
   <213> Homo sapiens
<400> 37
   Met Ala Ala Arg Gly Ser Gly Pro Arg Ala Leu Arg Leu Leu Leu Leu
   1               5                   10                  15
   Val Gln Leu Val Ala Gly Arg Cys Gly Leu Ala Gly Ala Ala Gly Gly
               20                  25                  30
   Ala Gln Arg Gly Leu Ser Glu Pro Ser Ser Ile Ala Lys His Glu Asp
           35                  40                  45
   Ser Leu Leu Lys Asp Leu Phe Gln Asp Tyr Glu Arg Trp Val Arg Pro
       50                  55                  60
   Val Glu His Leu Asn Asp Lys Ile Lys Ile Lys Phe Gly Leu Ala Ile
   65                  70                  75                  80
   Ser Gln Leu Val Asp Val Asp Glu Lys Asn Gln Leu Met Thr Thr Asn
                   85                  90                  95
   Val Trp Leu Lys Gln Glu Trp Ile Asp Val Lys Leu Arg Trp Asn Pro
               100                 105                 110
   Asp Asp Tyr Gly Gly Ile Lys Val Ile Arg Val Pro Ser Asp Ser Val
           115                 120                 125
   Trp Thr Pro Asp Ile Val Leu Phe Asp Asn Ala Asp Gly Arg Phe Glu
       130                 135                 140
   Gly Thr Ser Thr Lys Thr Val Ile Arg Tyr Asn Gly Thr Val Thr Trp
   145                 150                 155                 160
   Thr Pro Pro Ala Asn Tyr Lys Ser Ser Cys Thr Ile Asp Val Thr Phe
                   165                 170                 175
   Phe Pro Phe Asp Leu Gln Asn Cys Ser Met Lys Phe Gly Ser Trp Thr
               180                 185                 190
   Tyr Asp Gly Ser Gln Val Asp Ile Ile Leu Glu Asp Gln Asp Val Asp
           195                 200                 205
   Lys Arg Asp Phe Phe Asp Asn Gly Glu Trp Glu Ile Val Ser Ala Thr
       210                 215                 220
   Gly Ser Lys Gly Asn Arg Thr Asp Ser Cys Cys Trp Tyr Pro Tyr Val
   225                 230                 235                 240
   Thr Tyr Ser Phe Val Ile Lys Arg Leu Pro Leu Phe Tyr Thr Leu Phe
                   245                 250                 255
   Leu Ile Ile Pro Cys Ile Gly Leu Ser Phe Leu Thr Val Leu Val Phe
               260                 265                 270
   Tyr Leu Pro Ser Asn Glu Gly Glu Lys Ile Cys Leu Cys Thr Ser Val
           275                 280                 285
   Leu Val Ser Leu Thr Val Phe Leu Leu Val Ile Glu Glu Ile Ile Pro
       290                 295                 300
   Ser Ser Ser Lys Val Ile Pro Leu Ile Gly Glu Tyr Leu Val Phe Thr
   305                 310                 315                 320
   Met Ile Phe Val Thr Leu Ser Ile Met Val Thr Val Phe Ala Ile Asn
                   325                 330                 335
   Ile His His Arg Ser Ser Ser Thr His Asn Ala Met Ala Pro Leu Val
               340                 345                 350
   Arg Lys Ile Phe Leu His Thr Leu Pro Lys Leu Leu Cys Met Arg Ser
           355                 360                 365
   His Val Asp Arg Tyr Phe Thr Gln Lys Glu Glu Thr Glu Ser Gly Ser
       370                 375                 380
   Gly Pro Lys Ser Ser Arg Asn Thr Leu Glu Ala Ala Leu Asp Ser Ile
   385                 390                 395                 400
   Arg Tyr Ile Thr Arg His Ile Met Lys Glu Asn Asp Val Arg Glu Val
                   405                 410                 415
   Val Glu Asp Trp Lys Phe Ile Ala Gln Val Leu Asp Arg Met Phe Leu
               420                 425                 430
   Trp Thr Phe Leu Phe Val Ser Ile Val Gly Ser Leu Gly Leu Phe Val
           435                 440                 445
   Pro Val Ile Tyr Lys Trp Ala Asn Ile Leu Ile Pro Val His Ile Gly
       450                 455                 460
   Asn Ala Asn Lys
   465
<210> 38
   <211> 505
   <212> PRT
   <213> Homo sapiens
<400> 38
   Met Gly Ser Gly Pro Leu Ser Leu Pro Leu Ala Leu Ser Pro Pro Arg
   1               5                   10                  15
   Leu Leu Leu Leu Leu Leu Leu Ser Leu Leu Pro Val Ala Arg Ala Ser
               20                  25                  30
   Glu Ala Glu His Arg Leu Phe Glu Arg Leu Phe Glu Asp Tyr Asn Glu
           35                  40                  45
   Ile Ile Arg Pro Val Ala Asn Val Ser Asp Pro Val Ile Ile His Phe
       50                  55                  60
   Glu Val Ser Met Ser Gln Leu Val Lys Val Asp Glu Val Asn Gln Ile
   65                  70                  75                  80
   Met Glu Thr Asn Leu Trp Leu Lys Gln Ile Trp Asn Asp Tyr Lys Leu
                   85                  90                  95
   Lys Trp Asn Pro Ser Asp Tyr Gly Gly Ala Glu Phe Met Arg Val Pro
               100                 105                 110
   Ala Gln Lys Ile Trp Lys Pro Asp Ile Val Leu Tyr Asn Asn Ala Val
           115                 120                 125
   Gly Asp Phe Gln Val Asp Asp Lys Thr Lys Ala Leu Leu Lys Tyr Thr
       130                 135                 140
   Gly Glu Val Thr Trp Ile Pro Pro Ala Ile Phe Lys Ser Ser Cys Lys
   145                 150                 155                 160
   Ile Asp Val Thr Tyr Phe Pro Phe Asp Tyr Gln Asn Cys Thr Met Lys
                   165                 170                 175
   Phe Gly Ser Trp Ser Tyr Asp Lys Ala Lys Ile Asp Leu Val Leu Ile
               180                 185                 190
   Gly Ser Ser Met Asn Leu Lys Asp Tyr Trp Glu Ser Gly Glu Trp Ala
           195                 200                 205
   Ile Ile Lys Ala Pro Gly Tyr Lys His Asp Ile Lys Tyr Asn Cys Cys
       210                 215                 220
   Glu Glu Ile Tyr Pro Asp Ile Thr Tyr Ser Leu Tyr Ile Arg Arg Leu
   225                 230                 235                 240
   Pro Leu Phe Tyr Thr Ile Asn Leu Ile Ile Pro Cys Leu Leu Ile Ser
   245 250 255
   Phe Leu Thr Val Leu Val Phe Tyr Leu Pro Ser Asp Cys Gly Glu Lys
   260 265 270
   Val Thr Leu Cys Ile Ser Val Leu Leu Ser Leu Thr Val Phe Leu Leu
   275 280 285
   Val Ile Thr Glu Thr Ile Pro Ser Thr Ser Leu Val Ile Pro Leu Ile
   290 295 300
   Gly Glu Tyr Leu Leu Phe Thr Met Ile Phe Val Thr Leu Ser Ile Val
   305 310 315 320
   Ile Thr Val Phe Val Leu Asn Val His Tyr Arg Thr Pro Thr Thr His
   325 330 335
   Thr Met Pro Ser Trp Val Lys Thr Val Phe Leu Asn Leu Leu Pro Arg
   340 345 350
   Val Met Phe Met Thr Arg Pro Thr Ser Asn Glu Gly Asn Ala Gln Lys
   355 360 365
   Pro Arg Pro Leu Tyr Gly Ala Glu Leu Ser Asn Leu Asn Cys Phe Ser
   370 375 380
   Arg Ala Glu Ser Lys Gly Cys Lys Glu Gly Tyr Pro Cys Gln Asp Gly
   385 390 395 400
   Met Cys Gly Tyr Cys His His Arg Arg Ile Lys Ile Ser Asn Phe Ser
   405 410 415
   Ala Asn Leu Thr Arg Ser Ser Ser Ser Glu Ser Val Asp Ala Val Leu
   420 425 430
   Ser Leu Ser Ala Leu Ser Pro Glu Ile Lys Glu Ala Ile Gln Ser Val
   435 440 445
   Lys Tyr Ile Ala Glu Asn Met Lys Ala Gln Asn Glu Ala Lys Glu Ile
   450 455 460
   Gln Asp Asp Trp Lys Tyr Val Ala Met Val Ile Asp Arg Ile Phe Leu
   465 470 475 480
   Trp Val Phe Thr Leu Val Cys Ile Leu Gly Thr Ala Gly Leu Phe Leu
   485 490 495
   Gln Pro Leu Met Ala Arg Glu Asp Ala
   500 505
<210> 39
   <211> 498
   <212> PRT
   <213> Homo sapiens
<400> 39
   Met Arg Arg Ala Pro Ser Leu Val Leu Phe Phe Leu Val Ala Leu Cys
   1               5                   10                  15
   Gly Arg Gly Asn Cys Arg Val Ala Asn Ala Glu Glu Lys Leu Met Asp
               20                  25                  30
   Asp Leu Leu Asn Lys Thr Arg Tyr Asn Asn Leu Ile Arg Pro Ala Thr
           35                  40                  45
   Ser Ser Ser Gln Leu Ile Ser Ile Lys Leu Gln Leu Ser Leu Ala Gln
       50                  55                  60
   Leu Ile Ser Val Asn Glu Arg Glu Gln Ile Met Thr Thr Asn Val Trp
   65                  70                  75                  80
   Leu Lys Gln Glu Trp Thr Asp Tyr Arg Leu Thr Trp Asn Ser Ser Arg
                   85                  90                  95
   Tyr Glu Gly Val Asn Ile Leu Arg Ile Pro Ala Lys Arg Ile Trp Leu
               100                 105                 110
   Pro Asp Ile Val Leu Tyr Asn Asn Ala Asp Gly Thr Tyr Glu Val Ser
           115                 120                 125
   Val Tyr Thr Asn Leu Ile Val Arg Ser Asn Gly Ser Val Leu Trp Leu
       130                 135                 140
   Pro Pro Ala Ile Tyr Lys Ser Ala Cys Lys Ile Glu Val Lys Tyr Phe
   145                 150                 155                 160
   Pro Phe Asp Gln Gln Asn Cys Thr Leu Lys Phe Arg Ser Trp Thr Tyr
                   165                 170                 175
   Asp His Thr Glu Ile Asp Met Val Leu Met Thr Pro Thr Ala Ser Met
               180                 185                 190
   Asp Asp Phe Thr Pro Ser Gly Glu Trp Asp Ile Val Ala Leu Pro Gly
           195                 200                 205
   Arg Arg Thr Val Asn Pro Gln Asp Pro Ser Tyr Val Asp Val Thr Tyr
       210                 215                 220
   Asp Phe Ile Ile Lys Arg Lys Pro Leu Phe Tyr Thr Ile Asn Leu Ile
   225                 230                 235                 240
   Ile Pro Cys Val Leu Thr Thr Leu Leu Ala Ile Leu Val Phe Tyr Leu
                   245                 250                 255
   Pro Ser Asp Cys Gly Glu Lys Met Thr Leu Cys Ile Ser Val Leu Leu
               260                 265                 270
   Ala Leu Thr Phe Phe Leu Leu Leu Ile Ser Lys Ile Val Pro Pro Thr
           275                 280                 285
   Ser Leu Asp Val Pro Leu Ile Gly Lys Tyr Leu Met Phe Thr Met Val
       290                 295                 300
   Leu Val Thr Phe Ser Ile Val Thr Ser Val Cys Val Leu Asn Val His
   305                 310                 315                 320
   His Arg Ser Pro Ser Thr His Thr Met Ala Pro Trp Val Lys Arg Cys
                   325                 330                 335
   Phe Leu His Lys Leu Pro Thr Phe Leu Phe Met Lys Arg Pro Gly Pro
               340                 345                 350
   Asp Ser Ser Pro Ala Arg Ala Phe Pro Pro Ser Lys Ser Cys Val Thr
           355                 360                 365
   Lys Pro Glu Ala Thr Ala Thr Ser Thr Ser Pro Ser Asn Phe Tyr Gly
       370                 375                 380
   Asn Ser Met Tyr Phe Val Asn Pro Ala Ser Ala Ala Ser Lys Ser Pro
   385                 390                 395                 400
   Ala Gly Ser Thr Pro Val Ala Ile Pro Arg Asp Phe Trp Leu Arg Ser
                   405                 410                 415
   Ser Gly Arg Phe Arg Gln Asp Val Gln Glu Ala Leu Glu Gly Val Ser
               420                 425                 430
   Phe Ile Ala Gln His Met Lys Asn Asp Asp Glu Asp Gln Ser Val Val
           435                 440                 445
   Glu Asp Trp Lys Tyr Val Ala Met Val Val Asp Arg Leu Phe Leu Trp
       450                 455                 460
   Val Phe Met Phe Val Cys Val Leu Gly Thr Val Gly Leu Phe Leu Pro
   465                 470                 475                 480
   Pro Leu Phe Gln Thr His Ala Ala Ser Glu Gly Pro Tyr Ala Ala Gln
                   485                 490                 495
   Arg Asp
<210> 40
   <211> 31
   <212> DNA
   <213> Homo sapiens
<220>
   <221> variation
   <222> (16)..(16)
   <223> n= C or T
<400> 40
   gctatggatg ttcaanttgt gtgggaaata c 31
<210> 41
   <211> 31
   <212> DNA
   <213> Homo sapiens
<220>
   <221> variation
   <222> (16)..(16)
   <223> n= A, G or T
<400> 41
   tagaccaaag agccanttgc actccaggct a 31
<210> 42
   <211> 31
   <212> DNA
   <213> Homo sapiens
<220>
   <221> variation
   <222> (16)..(16)
   <223> n= C or T
<400> 42
   acctggggca ggttcnatcc cttgcagtta g 31
<210> 43
   <211> 31
   <212> DNA
   <213> Homo sapiens
<220>
   <221> variation
   <222> (16)..(16)
   <223> n= A, G, C or T
<400> 43
   cctcccttgc cagtcngcct gtcttcctcc c 31
<210> 44
   <211> 31
   <212> DNA
   <213> Homo sapiens
<220>
   <221> variation
   <222> (16)..(16)
   <223> n= A or G
<400> 44
   taattaagaa actaantcta gcaggctaaa g 31
<210> 45
   <211> 31
   <212> DNA
   <213> Homo sapiens
<220>
   <221> variation
   <222> (16)..(16)
   <223> n= A or G
<400> 45
   caatcattct gtccantcca gccaaggttc c 31
<210> 46
   <211> 31
   <212> DNA
   <213> Homo sapiens
<220>
   <221> variation
   <222> (16)..(16)
   <223> n= C or A
<400> 46
   accgaataac cgctgntaat atggcccccg c 31
<210> 47
   <211> 31
   <212> DNA
   <213> Homo sapiens
<220>
   <221> variation
   <222> (16)..(16)
   <223> n= C or T
<400> 47
   ctttctgttc tttttntttc ttctcacgct c 31
<210> 48
   <211> 31
   <212> DNA
   <213> Homo sapiens
<220>
   <221> variation
   <222> (16)..(16)
   <223> n=A or G
<400> 48
   tatgactcca ggaccnctat attttctcca g 31
<210> 49
   <211> 31
   <212> DNA
   <213> Homo sapiens
<220>
   <221> variation
   <222> (16)..(16)
   <223> n= A or G
<400> 49
   gaagatatga ctccangacc actatatttt c 31
<210> 50
   <211> 31
   <212> DNA
   <213> Homo sapiens
<220>
   <221> variation
   <222> (16)..(16)
   <223> n= A or G
<400> 50
   atgttctgac taatgnacta aggctcattg c 31
<210> 51
   <211> 31
   <212> DNA
   <213> Homo sapiens
<220>
   <221> variation
   <222> (16)..(16)
   <223> n= C or G
<400> 51
   tgtttgggaa ttttancaaa tgatggtgtt t 31
<210> 52
   <211> 31
   <212> DNA
   <213> Homo sapiens
<220>
   <221> variation
   <222> (16)..(16)
   <223> n= A or G
<400> 52
   cttgcagtgt ggcagncata acttctgatg c 31
<210> 53
   <211> 31
   <212> DNA
   <213> Homo sapiens
<220>
   <221> variation
   <222> (16)..(16)
   <223> n= C or T
<400> 53
   tgtggcaggc ataacntctg atgctaatgt t 31
<210> 54
   <211> 31
   <212> DNA
   <213> Homo sapiens
<220>
   <221> variation
   <222> (16)..(16)
   <223> n= C or A
<400> 54
   agctattgga catgcnggca cctgtttggg a 31
<210> 55
   <211> 31
   <212> DNA
   <213> Homo sapiens
<220>
   <221> variation
   <222> (16)..(16)
   <223> n= A or G
<400> 55
   tgtgtggaag attaanacgt ccatgaaggg t 31
<210> 56
   <211> 31
   <212> DNA
   <213> Homo sapiens
<220>
   <221> variation
   <222> (16)..(16)
   <223> n= C or G
<400> 56
   ttcgctacat tacaanacac atcatgaagg a 31
<210> 57
   <211> 31
   <212> DNA
   <213> Homo sapiens
<220>
   <221> variation
   <222> (16)..(16)
   <223> n= C or T
<400> 57
   gtcagacacg ttggcnacag gccggatgat c 31
<210> 58
   <211> 31
   <212> DNA
   <213> Homo sapiens
<220>
   <221> variation
   <222> (16)..(16)
   <223> n= C or T
<400> 58
   aggctgcaag gagggntacc cctgccagga c 31
<210> 59
   <211> 31
   <212> DNA
   <213> Homo sapiens
<220>
   <221> variation
   <222> (16)..(16)
   <223> n=G or A
<400> 59
   ttttgctcct aagggnacat ttaacaatgg a 31
<210> 60
   <211> 31
   <212> DNA
   <213> Homo sapiens
<220>
   <221> variation
   <222> (16)..(16)
   <223> n= C or T
<400> 60
   tcagccttag cactanggac attttgggct g 31
<210> 61
   <211> 31
   <212> DNA
   <213> Homo sapiens
<220>
   <221> variation
   <222> (16)..(16)
   <223> n= G or A
<400> 61
   tccacccagt ttatgntgta ctaagacagt t 31
<210> 62
   <211> 31
   <212> DNA
   <213> Homo sapiens
<220>
   <221> variation
   <222> (16)..(16)
   <223> n= C or A
<400> 62
   agcagcagca gcagcngccg cggcggcgac a 31
<210> 63
   <211> 31
   <212> DNA
   <213> Homo sapiens
<220>
   <221> variation
   <222> (16)..(16)
   <223> n= C or G
<400> 63
   agcagcagca gcagcngcgg cggcgacagc g 31
<210> 64
   <211> 31
   <212> DNA
   <213> Homo sapiens
<220>
   <221> variation
   <222> (16)..(16)
   <223> n= C or T
<400> 64
   agctagaagg cacccnttga ggaagaggac c 31

## Description

### Field of the Invention

The present invention relates to lung cancer, and more precisely to a method of testing a subject thought to be predisposed to lung cancer.

### Background of the invention

Lung cancer is the most common cause of cancer death worldwide with over 1 million cases annually. This cancer is caused predominantly by tobacco smoking, with cessation of tobacco consumption being the primary method for prevention. The risk among those who quit smoking remains elevated (although less than those who continue to smoke), and former smokers make up an increasing proportion of lung cancer patients in countries where tobacco consumption has declined.

Actually treatment strategies are of limited efficacy, with an overall 5-year survival rate of about 15%.

Since lung cancer has an important heritable component, then identifying genes that are involved in its etiology may suggest chemoprevention targets or allow for identification of groups at high risk.

WO2006/123955 discloses SNPs associated with the risk of developing lung cancer.

Nevertheless, and despite a large number of studies including both sporadic and multi-case families, success in identifying genes that cause lung cancer has been extremely limited.

### Summary of the invention

The present invention relates to a method of testing a human thought to be predisposed to having lung cancer which comprises the step of analyzing a biological sample from said human for detecting the presence of a polymorphism on chromosome 15q25 associated with lung cancer as defined in claims 1-3.

the polymorphism on chromosome 15q25 associated with lung cancer corresponds to a single nucleotide polymorphism (SNP) as defined in claim.

Said single nucleotide polymorphism is selected from the group comprising *rs17483548* (nucleotide N at position 16 of SEQ ID NO:1, wherein allele A is associated to lung cancer), *rs17405217* (nucleotide N at position 16 of SEQ ID NO:2, wherein allele T is associated to lung cancer), *rs17483721* (nucleotide N at position 16 of SEQ ID NO:3, wherein allele C is associated to lung cancer), *rs2656052* (nucleotide N at position 16 of SEQ ID NO:4, wherein allele G is associated to lung cancer), *rs2568494* (nucleotide N at position 16 of SEQ ID NO:5, wherein allele A is associated to lung cancer), *rs7181486* (nucleotide N at position 16 of SEQ ID NO:6, wherein allele C is associated to lung cancer), *rs17483929* (nucleotide N at position 16 of SEQ ID NO:7, wherein allele A is associated to lung cancer), *rs2656065* (nucleotide N at position 16 of SEQ ID NO:8, wherein allele T is associated to lung cancer), *rs2009746* (nucleotide N at position 16 of SEQ ID NO:9, wherein allele C is associated to lung cancer), *rs17484235* (nucleotide N at position 16 of SEQ ID NO:10, wherein allele G is associated to lung cancer), *rs1504550* (nucleotide N at position 16 of SEQ ID NO:11, wherein allele C is associated to lung cancer), *rs17484524* (nucleotide N at position 16 of SEQ ID NO:12, wherein allele G is associated to lung cancer), *rs9788721* (nucleotide N at position 16 of SEQ ID NO:13, wherein allele C is associated to lung cancer), *rs8034191* (nucleotide N at position 16 of SEQ ID NO:14, wherein allele C is associated to lung cancer), *rs10519203* (nucleotide N at position 16 of SEQ ID NO:15, wherein allele G is associated to lung cancer), *rs8031948* (nucleotide N at position 16 of SEQ ID NO:16, wherein allele T is associated to lung cancer), *rs931794* (nucleotide N at position 31 of SEQ ID NO:17, wherein allele G is associated to lung cancer), *rs2036527* (nucleotide N at position 31 of SEQ ID NO:18, wherein allele A is associated to lung cancer), *rs17486278* (nucleotide N at position 16 of SEQ ID NO:19, wherein allele C is associated to lung cancer), *rs7180002* (nucleotide N at position 16 of SEQ ID NO:20, wherein allele T is associated to lung cancer), *rs951266* (nucleotide N at position 16 of SEQ ID NO:21, wherein allele T is associated to lung cancer), *rs1317286* (nucleotide N at position 16 of SEQ ID NO:24, wherein allele G is associated to lung cancer), and *rs17487223* (nucleotide N at position 16 of SEQ ID NO:25, wherein allele T is associated to lung cancer).

The present disclosure also relates to kits that comprise, e.g., probes for identifying the polymorphism herein, e.g., packaged in suitable containers with instructions for correlating detected polymorphism to lung cancer predisposition.

Finally, the present disclosure also relates to a method of identifying modulators of lung cancer comprising the step of contacting a potential modulator to a protein selected in the group comprising IREB2, such as SEQ ID NO: 33, LOC123688, such as SEQ ID NO: 34 and SEQ ID NO:35, PSMA4, such as SEQ ID NO: 36, CHRNA5, such as SEQ ID NO: 37, CHRNA3, such as SEQ ID NO: 38, and CHRNB4, such as SEQ ID NO: 39, or to a nucleic acid that encodes such a protein. An effect of the potential modulator on the gene or gene product is detected, thereby identifying whether the potential modulator modulates lung cancer.

### Brief Description of the Drawings

Figure 1 shows (a) the QQ plot for bottom 90% of p-value and (b) top 10% of p-value, as well as (c) scatter plot of p-value in -log scale from the trend test for 310,023 genotyped variants comparing 1,926 lung cancer cases and 2,522 controls.
Figure 2 shows the lung cancer area of interest across 15q25.
Figure 3 shows the Odds Ratio (OR) and 95% confidence interval (CI) for lung cancer comparing heterozygous (T/C) and homozygous (C/C) genotypes of *rs8034191* to homozygous (T/T) genotype.
Figure 4 shows the relevance of smoking and of *rs8034191* genotype to cancer mortality depending of the subject age.
Figure 5 shows the OR and 95% CI for upper aero-digestive for the *rs8034191* variant.

### Detailed description

The present invention is based on the discovery by the present inventors that polymorphisms located on chromosome 15q25 are correlated with lung cancer predisposition.

Thus, in a first aspect, the present invention provides a method of testing a human thought to be predisposed to having lung cancer which comprises the step of analyzing a biological sample from said human for detecting the presence of a polymorphism on chromosome 15q25 associated with lung cancer as defined in the claims.

Said method, by detecting the presence of a polymorphism on chromosome 15q25 associated with lung cancer in a human enables to confirm that said human has or is predisposed for having lung cancer.

Preferably, a polymorphism on chromosome 15q25 corresponds to a polymorphism located between position 76,480,000 and 76,800,000 of the human chromosome 15 (NC_000015), preferably between position 76,499,754 and 76,795,005, and most preferably between position 76,517,000 and 76,711,000.

As used herein, the expression "biological sample" refers to solid tissues such as, for example, a lung biopsy; buccal swab, fluids and excretions such as for example, sputum, induced sputum, blood, serum, plasma, urine. Preferably, said biological sample is a fluid sample and most preferably a blood sample.

Typically, the polymorphism on chromosome 15q25 associated with lung cancer corresponds to a single nucleotide polymorphism (SNP), which corresponds to a mutation of a single base pair, which mutation can exist in the human population with a frequency inferior to 1%, to 0.1 %, to 0.01 %, and even inferior to 0.001%.

Preferably, said single nucleotide polymorphism is selected from the group comprising *rs17483548* (nucleotide N at position 16 of SEQ ID NO:1, wherein allele A is associated to lung cancer), *rs17405217* (nucleotide N at position 16 of SEQ ID NO:2, wherein allele T is associated to lung cancer), *rs17483721* (nucleotide N at position 16 of SEQ ID NO:3, wherein allele C is associated to lung cancer), *rs2656052* (nucleotide N at position 16 of SEQ ID NO:4, wherein allele G is associated to lung cancer), *rs2568494* (nucleotide N at position 16 of SEQ ID NO:5, wherein allele A is associated to lung cancer), *rs7181486* (nucleotide N at position 16 of SEQ ID NO:6, wherein allele C is associated to lung cancer), *rs17483929* (nucleotide N at position 16 of SEQ ID NO:7, wherein allele A is associated to lung cancer), *rs2656065* (nucleotide N at position 16 of SEQ ID NO:8, wherein allele T is associated to lung cancer), *rs2009746* (nucleotide N at position 16 of SEQ ID NO:9, wherein allele C is associated to lung cancer), *rs17484235* (nucleotide N at position 16 of SEQ ID NO: 10, wherein allele G is associated to lung cancer), *rs1504550* (nucleotide N at position 16 of SEQ ID NO:11, wherein allele C is associated to lung cancer), *rs17484524* (nucleotide N at position 16 of SEQ ID NO:12, wherein allele G is associated to lung cancer), *rs9788721* (nucleotide N at position 16 of SEQ ID NO:13, wherein allele C is associated to lung cancer), *rs8034191* (nucleotide N at position 16 of SEQ ID NO:14, wherein allele C is associated to lung cancer), *rs10519203* (nucleotide N at position 16 of SEQ ID NO:15, wherein allele G is associated to lung cancer), *rs8031948* (nucleotide N at position 16 of SEQ ID NO:16, wherein allele T is associated to lung cancer), *rs931794* (nucleotide N at position 31 of SEQ ID NO:17, wherein allele G is associated to lung cancer), *rs2036527* (nucleotide N at position 31 of SEQ ID NO:18, wherein allele A is associated to lung cancer), *rs17486278* (nucleotide N at position 16 of SEQ ID NO:19, wherein allele C is associated to lung cancer), *rs7180002* (nucleotide N at position 16 of SEQ ID NO:20, wherein allele T is associated to lung cancer), *rs951266* (nucleotide N at position 16 of SEQ ID NO:21, wherein allele T is associated to lung cancer), *rs16969968* (nucleotide N at position 16 of SEQ ID NO:22, wherein allele A is associated to lung cancer), *rs1051730* (nucleotide N at position 31 of SEQ ID NO:23, wherein allele A is associated to lung cancer), *rs1317286* (nucleotide N at position 16 of SEQ ID NO:24, wherein allele G is associated to lung cancer), and *rs17487223* (nucleotide N at position 16 of SEQ ID NO:25, wherein allele T is associated to lung cancer).

The single nucleotide polymorphism on chromosome 15q25 associated with lung cancer may also include a single nucleotide polymorphism selected in the group comprising rs7177092 (nucleotide N at position 16 of SEQ ID NO:40), rs8032410 (nucleotide N at position 16 of SEQ ID NO:41), rs2055588 (nucleotide N at position 16 of SEQ ID NO:42), rs954144 (nucleotide N at position 16 of SEQ ID NO:43), rs8033501(nucleotide N at position 16 of SEQ ID NO:44), rs3743080 (nucleotide N at position 16 of SEQ ID NO:45), rs3813571 (nucleotide N at position 16 of SEQ ID NO:46), rs1065640 (nucleotide N at position 16 of SEQ ID NO:47), rs11551787 (nucleotide N at position 16 of SEQ ID NO:48), rs11551783 (nucleotide N at position 16 of SEQ ID NO:49), rs1155782 (nucleotide N at position 16 of SEQ ID NO:50), rs11551779 (nucleotide N at position 16 of SEQ ID NO:51), rs11551781 (nucleotide N at position 16 of SEQ ID NO:52), rs11551786 (nucleotide N at position 16 of SEQ ID NO:53), rs11551784 (nucleotide N at position 16 of SEQ ID NO:54), rs1052040 (nucleotide N at position 16 of SEQ ID NO:55), rs1042500 (nucleotide N at position 16 of SEQ ID NO:56), rs8040868 (nucleotide N at position 16 of SEQ ID NO:57), rs1051731 (nucleotide N at position 16 of SEQ ID NO:58), rs660652 (nucleotide N at position 16 of SEQ ID NO:59), rs472054 (nucleotide N at position 16 of SEQ ID NO:60), rs8029939 (nucleotide N at position 16 of SEQ ID NO:61), rs12906406 (nucleotide N at position 16 of SEQ ID NO:62), rs12906525 (nucleotide N at position 16 of SEQ ID NO:63), and r s12904278 (nucleotide N at position 16 of SEQ ID NO:64).

Most preferably, said polymorphism is a single nucleotide polymorphism on chromosome 15q25 associated with lung cancer, wherein said single nucleotide polymorphism is selected from the group comprising *rs2656052* (nucleotide N at position 16 of SEQ ID NO:4, wherein allele G is associated to lung cancer), *rs17484235* (nucleotide N at position 16 of SEQ ID NO:10, wherein allele G is associated to lung cancer), *rs8034191* (nucleotide N at position 16 of SEQ ID NO:14, wherein allele C is associated to lung cancer), *rs10519203* (nucleotide N at position 16 of SEQ ID NO:15, wherein allele G is associated to lung cancer), *rs8031948* (nucleotide N at position 16 of SEQ ID NO:16, wherein allele T is associated to lung cancer), *rs931794* (nucleotide N at position 31 of SEQ ID NO:17, wherein allele G is associated to lung cancer), *rs2036527* (nucleotide N at position 31 of SEQ ID NO:18, wherein allele A is associated to lung cancer), *rs16969968* (nucleotide N at position 16 of SEQ ID NO:22, wherein allele A is associated to lung cancer), and *rs1317286* (nucleotide N at position 16 of SEQ ID NO:24, wherein allele G is associated to lung cancer.

Typical techniques for detecting a polymorphism may include restriction fragment length polymorphism, hybridization techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridization, ligation chain reaction, mini-sequencing, DNA"chips", allele-specific oligonucleotide hybridization with single or dual-labeled probes merged with PCR or with molecular beacons, and others.

Preferably, said technique for detecting a polymorphism is selected in the group comprising methods for detecting single nucleotide polymorphisms.

Depending on the polymorphism, the technique used for its detection can also be based on the analysis of mRNA transcript from a gene located on chromosome 15q25, if said polymorphism is also present in said mRNA sequence. As an example, said mRNA transcript is selected in the group comprising IREB2 mRNA, such as SEQ ID NO: 26, LOC123688 mRNA, such as SEQ ID NO: 27 and SEQ ID NO: 28, PSMA4 mRNA, such as SEQ ID NO: 29, CHRNA5 mRNA, such as SEQ ID NO: 30, CHRNA3 mRNA, such as SEQ ID NO: 31 , and CHRNB4 mRNA, such as SEQ ID NO: 32. As an example of a single nucleotide polymorphism on chromosome 15q25 associated with lung cancer which is also present in the mRNA sequence, one can cite *rs16969968* and *rs1051730.* This polymorphism analysis can be assessed by preparing mRNA/cDNA from cells in a biological sample from a human, and hybridizing the mRNA/cDNA with a reference polynucleotide. The prepared mRNA/cDNA can be used in hybridization or amplification assays that include, but are not limited to, polymerase chain reaction analyses, such as quantitative PCR (TaqMan), and probes arrays such as GeneChip^{™} DNA Arrays (AFFYMETRIX).

Still depending on the polymorphism, the technique used for its detection can also be based on the analysis of the protein translated from a gene located on chromosome 15q25, if said polymorphism is also present in said protein sequence. As an example, said protein is selected in the group comprising IREB2, such as SEQ ID NO: 33, LOC123688, such as SEQ ID NO: 34 and SEQ ID NO:35, PSMA4, such as SEQ ID NO: 36, CHRNA5, such as SEQ ID NO: 37, CHRNA3, such as SEQ ID NO: 38, and CHRNB4, such as SEQ ID NO: 39. As an example of a single nucleotide polymorphism on chromosome 15q25 associated with lung cancer which is also present in the protein sequence, one can cite *rs16969968* (substitution of aspartic acid (D) to asparagine (B) at amino acid position 398 (D398N) of the CHRNA5 protein). This polymorphism analysis can be assessed using an antibody (*i*., a radio-labeled, chromophore-labeled, fluorophore-labeled, or enzyme-labeled antibody), an antibody derivative (*e.g*., an antibody conjugate with a substrate or with the protein or ligand of a protein of a protein/ligand pair (*e.g*., biotin-streptavidin), or an antibody fragment (*e.g*., a single-chain antibody, an isolated antibody hypervariable domain, *etc.*) which binds or does not bind specifically to the protein comprising the analyzed polymorphism.

Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA).

Polyclonal antibodies can be prepared by immunizing a suitable animal, such as mouse, rabbit or goat, with a protein comprising the analyzed polymorphism, translated from a gene located on chromosome 15q25, or a fragment thereof. The antibody titer in the immunized animal can be monitored over time by standard techniques, such as with an ELISA using immobilized polypeptide. At an appropriate time after immunization, *e.g.,* when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed. , John Wiley & Sons, New York, 1994). Hybridoma cells producing the desired monoclonal antibody are detected by screening the hybridoma culture supernatants for antibodies that bind the polypeptide of interest, e.g., using a standard ELISA.

In a second aspect, the present disclosure concerns a kit that comprise, e.g., probes for identifying the polymorphism herein, e.g., packaged in suitable containers with instructions for correlating detected polymorphism to lung cancer predisposition.

In a third aspect, the present disclosure concerns a method of identifying modulators of lung cancer comprising the step of contacting a potential modulator to a protein selected in the group comprising IREB2, such as SEQ ID NO: 33, LOC123688, such as SEQ ID NO: 34 and SEQ ID NO:35, PSMA4, such as SEQ ID NO: 36, CHRNA5, such as SEQ ID NO: 37, CHRNA3, such as SEQ ID NO: 38, and CHRNB4, such as SEQ ID NO: 39, or to a nucleic acid that encodes such a protein. An effect of the potential modulator on the gene or gene product is detected, thereby identifying whether the potential modulator modulates lung cancer.

The gene or gene product that is contacted by the modulator can include any allelic form known for any of the *IREB2, LOC123688, PSMA4, CHRNA5, CHRNA3* or the *CHRNB4* gene. Any allelic forms, whether genes, RNAs or proteins, that positively correlate to lung cancer are preferred targets for modulator screening.

Effects of interest that can be screened for include: (a) increased or decreased expression of any of the *IREB2, LOC123688, PSMA4, CHRNA5, CHRNA3* or the *CHRNB4* gene, and/or any protein encoded by these genes, in the presence of a modulator; (b) a change in the timing or location of expression of any of the *IREB2, LOC123688, PSMA4, CHRNA5, CHRNA3* or the *CHRNB4* gene, and/or any protein encoded by these genes, in the presence of the modulator; (c) a change in any activity (e.g. increased or decreased activity) of the gene product of any of the *IREB2, LOC123688, PSMA4, CHRNA5, CHRNA3* or the *CHRNB4* gene in the presence of a modulator; and/or (d) a change in localization of proteins encoded by any of the *IREB2, LOC123688, PSMA4, CHRNA5, CHRNA3* or the *CHRNB4* gene in the presence of the modulator.

The precise format of the modulator screen will, of course, vary, depending on the effect(s) being detected and the equipment available. Northern analysis, quantitative RT- PCR and/or array-based detection formats can be used to distinguish expression levels or patterns of genes noted above. Protein expression levels can also be detected using available methods, such as western blotting, ELISA analysis, antibody hybridization, BIAcore, or the like. Any of these methods can be used to distinguish changes in expression levels of any of the *IREB2, LOC123688, PSMA4, CHRNA5, CHRNA3* or the *CHRNB4* gene, or the RNA or proteins encoded therein that result from a potential modulator.

Accordingly, one may screen for potential modulators of any of the *IREB2, LOC123688, PSMA4, CHRNA5, CHRNA3* or the *CHRNB4* gene and/or the RNA and protein encoded therein for activity or expression. For example, potential modulators (small molecules, RNAs (e.g., RNAi), organic molecules, inorganic molecules, proteins, hormones, transcription factors, or the like) can be contacted to a cell comprising an allele of interest and an effect on activity or expression (or both) of a gene, RNA or protein corresponding to any of the *IREB2, LOC123688, PSMA4, CHRNA5, CHRNA3* or the *CHRNB4* gene. For example, expression of any of the *IREB2, LOC123688, PSMA4, CHRNA5, CHRNA3* or the *CHRNB4* gene can be detected, e.g., via Northern analysis or quantitative (optionally real time) RT-PCR, before and after application of potential expression modulators. Similarly, promoter regions of the various genes (e.g., generally sequences in the region of the start site of transcription, e.g., within 5 kb of the start site, e.g., 1kb, or less e.g., within 500bp or 250bp or 100 bp of the start site) can be coupled to reporter constructs (CAT, beta-galactosidase, luciferase or any other available reporter) and can be similarly tested for expression activity modulation by the potential modulator. In either case, the assays can be performed in a high-throughput fashion, e.g., using automated fluid handling and/or detection systems, in serial or parallel fashion. Similarly, activity modulators can be tested by contacting a potential modulator to an appropriate cell using any of the activity detection methods herein, regardless of whether the activity that is detected is the result of activity modulation, expression modulation or both. These assays can be in vitro, cell-based, or can be screens for modulator activity performed on laboratory animals such as knock-out transgenic mice comprising a gene of interest.

Whole animal assays can also be used to assess the effects of modulators on cells or whole animals (e.g., transgenic knock-out mice), e.g., by monitoring an effect on a cell-based phenomenon, a change in displayed animal phenotype, or the like.

Potential modulator libraries to be screened for effects on expression and/or activity are available. These libraries can be random, or can be targeted. For example, a modulator library may be screened for effects on expression of any of the *IREB2, LOC123688, PSMA4, CHRNA5, CHRNA3* or the *CHRNB4* gene.

Targeted libraries include those designed using any form of a rational design technique that selects scaffolds or building blocks to generate combinatorial libraries. These techniques include a number of methods for the design and combinatorial synthesis of target-focused libraries, including morphing with bioisosteric transformations, analysis of target-specific privileged structures, and the like. In general, where information regarding structure of any of the *IREB2, LOC123688, PSMA4, CHRNA5, CHRNA3* or the *CHRNB4* gene or gene products is available, likely binding partners can be designed, e.g., using flexible docking approaches, or the like. Similarly, random libraries exist for a variety of basic chemical scaffolds. In either case, many thousands of scaffolds and building blocks for chemical libraries are available, including those with polypeptide, nucleic acid, carbohydrate, and other backbones. Commercially available libraries and library design services include those offered by CHEMICAL DIVERSITY (San Diego, CA), AFFYMETRIX (Santa Clara, CA), SIGMA (St. Louis MO), CHEMBRIDGE RESEARCH LABORATORIES (San Diego, CA), TIMTEC (Newark, DE), NUEVOLUTION A/S (Copenhagen, Denmark) and many others.

The present invention may be better understood by reference to the following nonlimiting Examples, which are provided as exemplary of the invention. The following examples are presented in order to more fully illustrate the preferred embodiments of the invention. They should in no way be construed, however, as limiting the broad scope of the invention.

### EXAMPLES

### 1) Genome wide analysis

We conducted a genome-wide association study of lung cancer using the ILLUMINA SENTRIX® HumanHap300 BeadChip that contains 317,139 SNPs and is estimated to tag approximately 80% of common genomic variation (BARRETT & CARDON, Nat. Genet., vol.38, p:659-662, 2006).

We initially genotyped 1989 cases and 2625 controls from the IARC central Europe lung cancer study.

The IARC study of lung cancer in central Europe was conducted with cancer institutes in 6 countries including Czech Republic (Prague, Olomouc, Brno), Hungary (Borsod, Heves, Szabolcs, Szolnok, Budapest), Poland (Warsaw, Lodz), Romania (Bucharest), Russia (Moscow) and Slovakia (Banska Bystrica, Bratislava, Nitra) between 1999 and 2002.

Briefly, each centre followed an identical protocol and was responsible for recruiting a consecutive group of newly diagnosed cases of lung cancer and a comparable group of hospital or population controls. All subjects were interviewed based on a standard questionnaire and information on lifestyle risk factors, occupational history; medical and family history has been collected. Written consent for participation was obtained from all study subjects and ethical approval has been obtained for all study centers as well as at IARC, the coordinating center. Controls in all centers except Warsaw were chosen among subjects admitted as in-patients or out-patients in the same hospital as the cases with conditions unrelated to tobacco including minor surgical conditions, benign disorders, common infections, eye conditions (except cataract or diabetic retinopathy) and common orthopeadic diseases (except osteoporosis). In Warsaw, population controls were selected by random sampling from the Polish Electronic List of Residents. This resulted in a total of 3052 potential controls. Cases and controls were frequency matched by sex, age (+/- 3 years), center, referral (or of residence) area and period of recruitment (+/- 6 months). The participation rates for both cases and controls were over 80% in all centers. Blood samples were collected from all subjects and DNA has been extracted. Candidate gene studies based on TAQMAN genotyping have been completed on over 100 candidate gene variants (BRENNAN et al., Hum. Mol. Genet., vol.16, 2007). 1989 lung cancer cases (434 adenocarcinoma, 815 squamous cell, 298 small cell and 379 of mixed cell or other histology) and 2625 controls had sufficient DNA amount and quality and was genotyped at Centre National Genotypage (CNG) with HUMANHAP300K BEADCHIP using the ILLUMINA INFINIUM PLATFORM, which included 317,139 SNPs (http://www.illumina.com/downloads/HUMAN HAP300Datasheet.pdf).

A systematic quality control was conducted on the raw ILLUMINA HUMANHAP300K genotyping data. This quality control has resulted in the exclusion of:
a) 6,107 variants with a genotype call rate of less than 95% were excluded, such as 50 individuals where the overall genotype completion rate was less than 90%.
b) 972 variants as their genotype distributions clearly deviated from that expected by HARDY-WEINBERG Equilibrium (HWE) among controls (p-value of less than 10⁻⁷).
c) 37 monomorphic variants.
d) 42 individuals as they showed sex discrepancies based on the heterozygosity rate from chromosome X.
e) 17 unexpected duplicates and 23 unexpected first degree relatives based on similarity of genotype.
f) 29 individuals where the HumanHap300K Illumina genotyping and Taqman genotyping from a previous candidate gene study (BRENNAN et al., abovementioned, 2007) of 40 overlapping variants showed >5% discordance.

The population outliers were detected using a set of 8,155 markers with low linkage disequilibrium (R²<0.001) to classify the subjects by Structure Association (FALUSH et al., Genetics, vol.164 (4), p: 1567-87, 2003). The data from the founders of the HapMap trios of YRI (N=60), CEU (n=60) and Asian (CHB/JPT combined); n=90) were used as the internal controls for this analysis assuming admixture model (individuals can have a mixed ancestry). 5 outliers were identified and excluded from the analysis. A comparison of 310,023 SNPs between 1926 cases and 2522 controls were used in the final analysis.

### 2) Genome wide statistical analysis

Each of the 310,023 SNPs was analyzed individually comparing common homozygotes to heterozygous and homozygous carriers of the variant allele by calculating p-values for trend (p_{trend}) in a logistic regression model and incorporating additional parameters including, country, age and sex.

For this analysis, the association between the 310,023 variants and the disease risk was estimated by the OR and 95% CI using the multivariate unconditional logistic regression using PLINK (PURCELL et al., Am J Hum. Genet., vol.81, p:559-575, 2007) assuming a co-dominant genetic model (the effect of the variant by log-additive model with 1 degree of freedom). Study matching variables of age, sex, and country of recruitment were included in the regression as covariates. The results that obtained a level of significance of p< 5X10⁻⁷ were considered significant at a genome wide level.

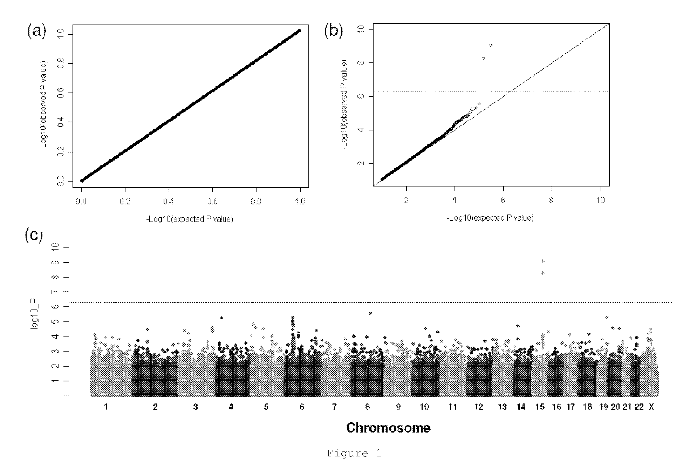

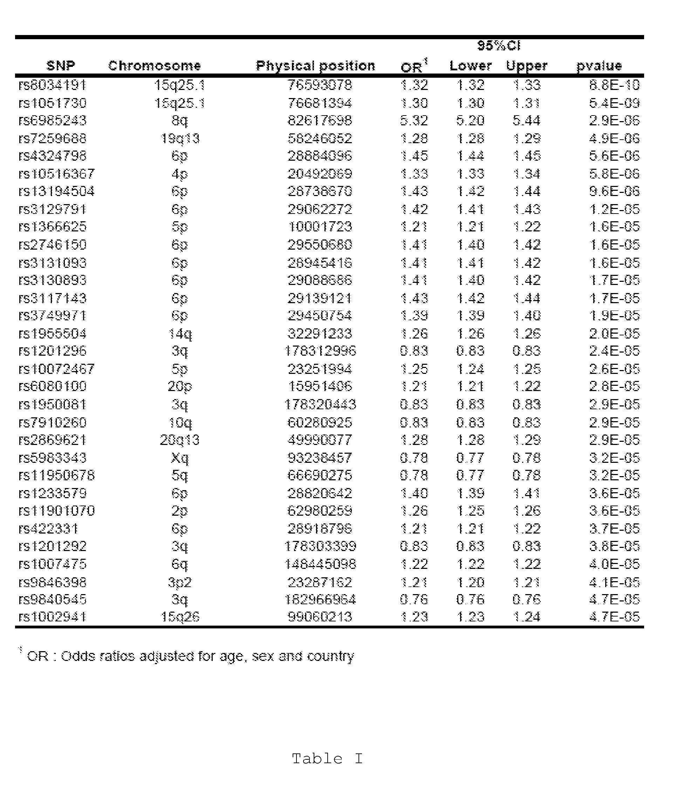

The figure 1 shows the (a) QQ plot for bottom 90% of p-values and (b) the top 10% of p-values, as well as (c) the scatter plot of p-values in -log scale from the trend test for the 310,023 genotyped variants comparing 1,926 lung cancer cases and 2,522 controls.

The results show that the distribution of the bottom 90% of p-values were similar to the expected distribution and the genomic control parameter was 1.03 implying that there was no systematic increase in false positive findings due to population stratification or any other form of bias (Figure 1a). However, the results established that there was a strong deviation between the observed and expected p-values among the top 10% (Figure 1b). In particular, two SNPs on chromosome 15q25, rs1051730 and rs8034191, were strongly associated with disease (p=5x10⁻⁹ and p=9x10⁻¹⁰ respectively) exceeding the genome-wide significance level of 5x10⁻⁷ (Figure 1c).

The principal components analysis implemented in the computer program EIGENSTRAT was used to control for potential effects of population stratification (PRICE et al., Nat. Genet., vol.38, p: 904-909, 2006). Adjustment of the logistic regression with principal components marginally decreased the significance of the disease associations for these markers (p=7x10⁻⁹ and p=1x10⁻⁹, respectively). Additional analyses were undertaken with potential outliers eliminated based on the principal components analysis, and again did not significantly modify the conclusions.

The Table I identified the markers with p_{trend}<5x10⁻⁵ in the genome-wide association study.

The results have indicated that population stratification was unlikely to account for the observation of the strong association with the 15q25 markers, and no other region contained markers exceeding the genome-wide significance level. However, the results show that several regions contained SNPs with significance that exceeded 5x10⁻⁵ (see Table I).

### 3) Association of 15q25 markers with lung cancer in IARC cohort

The association was confirmed by genotyping 34 additional 15q25 markers that were selected as follows.

First, the imputation methods proposed by ABECASIS and colleagues (http://www.sph.umich.edu/csg/abecasis/MACH/index.html) was used to identify additional genetic variants from the CEU Hapmap data that are likely to have a strong disease association, but that are not present in the HumanHap 300 panel. It was attempted to genotype the SNPs from the 15q25 region for which the p-value of the association statistic obtained from the imputed data was <10⁻⁶.

Second, SNPs of *CHRNA5* and *CHRNA3* that had been included in a previous study of these genes in nicotine dependence (SACCONE et al., Hum. Mol. Genet., vol.16, p: 36-49, 2007) were included.

Third, it was also attempted to genotype all non-synonymous SNPs in dbSNP from the six genes within or near the association region.

Follow up genotyping for the study was performed using the 5' exonuclease assay (TAQMAN, APPLIED BIOSYSTEMS) at the IARC and TAQMAN and AMPLIFLUOR at CNG. Cases and controls were randomly mixed when genotyped and laboratory personnel were blinded to case/control status. A randomly selected 7% of the study subjects (both cases and controls) and re-genotyped for each polymorphism to examine the reliability of the genotyping assays. Internal duplicate concordance was >99.9% and genotyping success rate 97% or greater. Genotype distributions did not depart from HARDY-WEINBERG Equilibrium (HWE) in the controls. The concordance between the ILLUMINA and TAQMAN genotyping for rs8034191 was greater than 99.8% in the IARC central Europe study.

The replication analysis was conducted using SAS 9.1 software. The association between the variants and the disease risk was estimated by the OR and 95% CI using the multivariate unconditional logistic regression estimated the effect of the variant by co-dominant and genotype-specific models. Stratification has been done by smoking status and histological subtypes to evaluate the effect modification by smoking status and the effect heterogeneity across histological subtypes. To evaluate whether there are any subtle differences among central Europeans, the principal component analysis of the ILLUMINA HUMANHAP300K genotyping data was conducted and included the eigen values of the first three axes. This analysis was conduced using EIGENSTRAT (PRICE et al., abovementioned, 2006). Briefly, this analysis consisted of two steps: (i) conducting principal components analysis to reduce the data into 3 axes, which refer to population subgroups (or ancestry); (ii) computing the association statistics adjusted for the inferred ancestry by including the eigen value into the logistic regression. As the results were almost identical with and without eigen values, it has been chosen to report the results without such eigen values adjustment.

The results for all markers tested in the 15q25 region, including those in the HumanHap 300 panel, are shown in Table II. In this table, the closest gene is indicated for markers within or near a gene. HapMap frequencies are provided when available. Finally, ORs and 95% CIs were calculated for polymorphic markers.

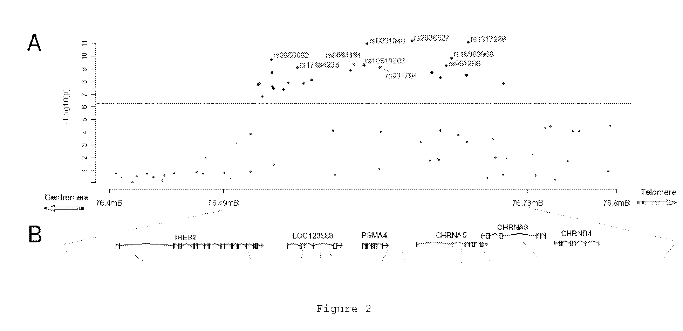

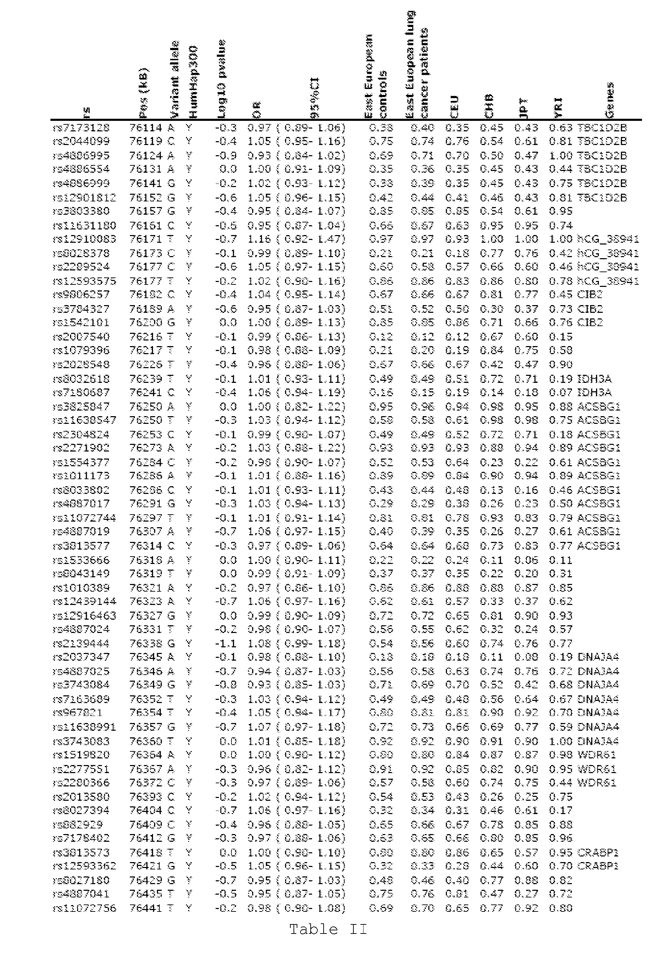

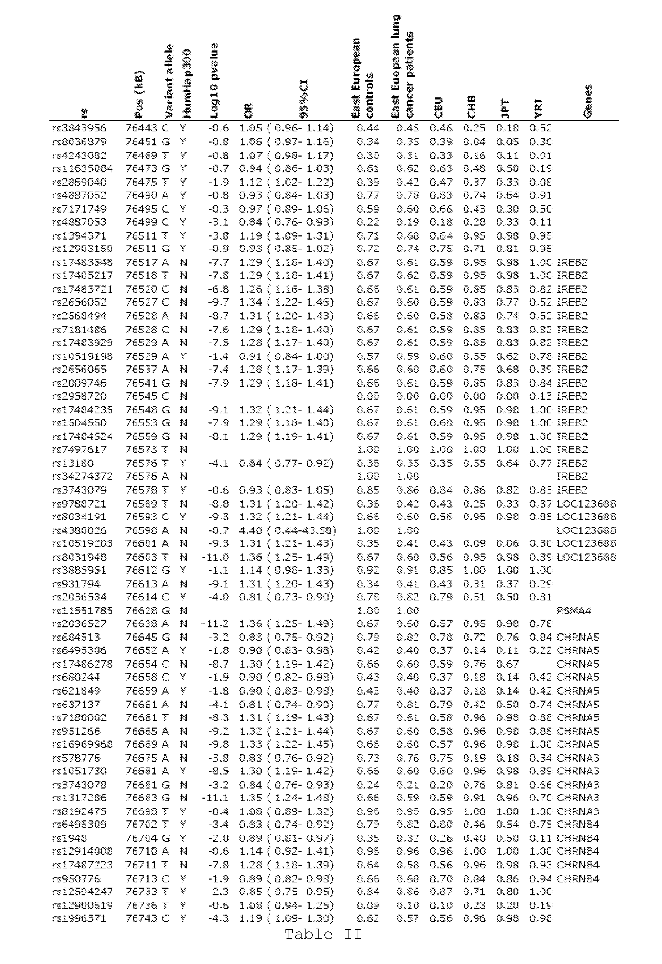

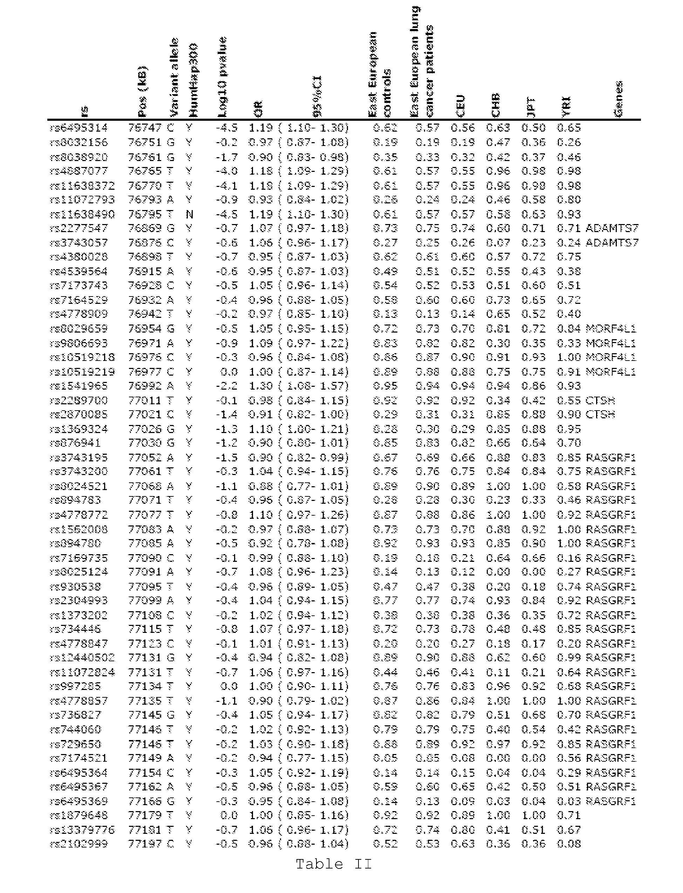

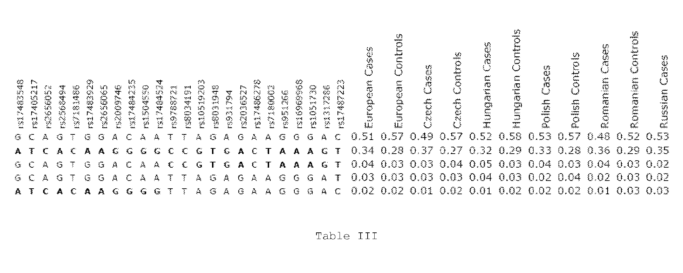

The figure 2 shows the lung cancer area of interest across 15q25. In this figure, the panel A shows p-values for SNPs genotyped in the 15q25 region (76.4-76.8 Mb). The dotted line indicates the threshold of p<5X10⁻⁷ at which results were considered genome-wide significant. Points labeled with "rs" numbers have a p<1X10⁻⁹. The panels B indicates the positions of the 6 known genes in the high LD genomic region approximately delineated by rs4887053 (76.49 Mb) and rs12594247 (76.73 Mb), which contains the SNPs strongly associated with lung cancer risk.

The Table III show the five common haplotypes (frequency > 1%) across the 24 15q25 SNPps which achieved genome wide significance in the central Europe IARC study. The haplotype frequencies are shown combined and individually for each participating country. The alleles associated with increased risk (OR>1) are indicated in bold. It has to be noticed that the two predominant haplotypes account for 81 % of the haplotypes in the central Europe IARC study and that three rare recombinant haplotypes are also present at frequencies > 1%.

The results established that twenty five of the additional genotyped markers showed evidence of association exceeding the genome-wide significance level of 5x10⁻⁷ (see figure 2). These markers span more than 182 kb but are in strong linkage disequilibrium (pairwise D'>0.8 and r2>0.6) with two predominant haplotypes accounting for more than 89% of the haplotypes in patients and controls (see Table III).

### 4) Association of 15q25 markers with lung cancer in other cohorts

In order to confirm the previous association, one of the principal disease associated SNPs (rs8034191) has been genotyped in 5 further independent studies of lung cancer. These were the EPIC cohort study (781 cases and 1578 controls), the CARET cohort study (764 cases and 1515 controls), the HUNT and Tromso cohort studies (235 cases and 392 controls), the Liverpool lung cancer case-control study (408 cases and 814 controls), and the Toronto lung cancer case-control study (330 cases and 453 controls).

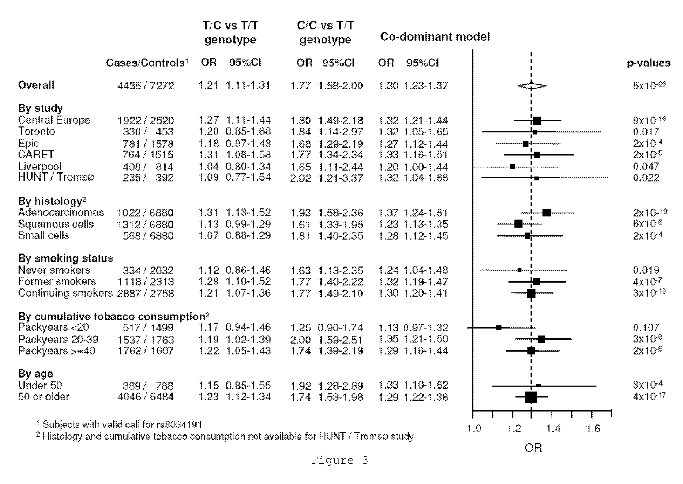

The figure 3 shows the OR and 95% CI for lung cancer comparing heterozygous (T/C) and homozygous (C/C) genotypes of rs8034191 to homozygous (T/T) genotype. The ORs are standardized by age, sex and country, and the overall OR is shown by the broken vertical line. Finally, the P-values are derived from the co-dominant model.

The results established that a similar increased risk is observed for both heterozygous and homozygous variants of rs8034191 in all 5 replication studies to that observed in the central Europe study (see figure 3). After pooling across all 6 studies, the ORs and 95% CIs were 1.21 (1.11-1.31) and 1.77 (1.58-2.00) for heterozygous and homozygous carriers respectively, the allelic OR was 1.30 (1.23-1.37) and the p-value for trend was 5x10⁻²⁰. Adjustment for cumulative tobacco consumption (packyears) had little effect on these estimates (allelic OR=1.26, 1.18-1.34). There was no statistical evidence of heterogeneity in the increase in risk across the 6 studies and there were no major differences between the main histological subtypes of lung cancer. An increased risk was apparent for never smokers, former smokers and current smokers. A similar risk was observed after stratifying by age at diagnosis and smoking intensity (see figure 3). The prevalence of the variant allele was 34% resulting in 66% of the control participants carrying at least one copy, and the percentage of lung cancer explained by carrying at least one allele (i.e. the population attributable risk) was 16%.

### 5) Association of 15q25 markers with lung cancer death

It has been previously estimated that the cumulative risk of lung cancer death among men in the 6 central European countries to be about 1% in never smokers, 5% among men who have quit smoking and 16% among continuing smokers (BRENNAN et al., Am. J. Epi., vol.164, p:1233-1241, 2006).

To illustrate the strong impact of the 15q25 locus, we have calculated cumulative risks for different genotypes in smokers and former smokers compared to non-smokers, using Polish men as a representative example. These calculations of cumulative risk have involved combining national lung cancer mortality rates with the relative risks associated with various smoking patterns, and with the prevalence of those patterns among controls (BRENNAN et al., abovementioned, 2006).

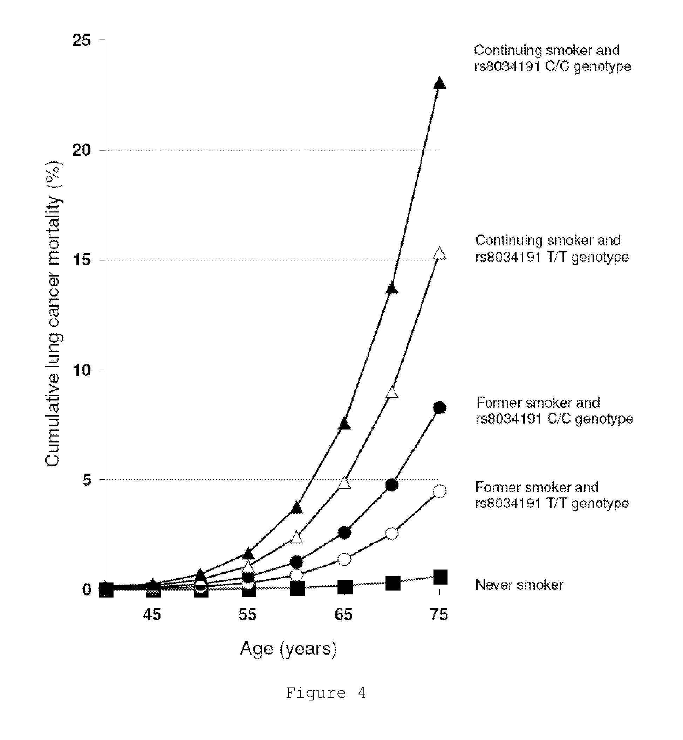

The figure 4 shows the relevance of smoking and of rs8034191 genotype to lung cancer mortality in men aged 45-75 years. Cumulative risk (in the absence of other causes of death) based on national lung cancer death rates for men in Poland in the year 2000, assuming that the prevalence of smoking, former smoking, and never smoking are as in this study and that the relative risks for lung cancer incidence and mortality are similar.

The results show that the cumulative risks of lung cancer death by age 75 are about 14% and 23% among smokers with the common and minor homozygous genotype respectively (see figure 4). Similarly, among former smokers the cumulative risks are about 4% and 8% respectively. Interestingly, the risk haplotype is rare in the Asian (Japanese and Chinese) and not observed in African (Yoruba) data in the HapMap database and many of the risk alleles have varied allele frequencies (see Table II), which implicate a different contribution of the 15q25 locus to lung cancer risk in these ethnic groups.

### 6) Association of 15q25 markers with lung cancer is specific

It has been further investigated whether the locus was associated with cancers of the head and neck including those of the oral cavity, larynx, pharynx and esophagus.

For this purpose, rs8034191 was analyzed in two separate studies of head and neck cancer conducted in Europe, the first being conducted in 5 countries of central Europe (i.e., Russia, Czech Republic, Romania, Hungary and Poland) and overlapping with the lung cancer controls from 5 of the 6 countries included in the present genome-wide association study (726 cases including 238 oral cavity/pharynx, 312 larynx and 156 esophagus and 20 with an overlapping site, and 694 controls) and the second study being conducted in 8 countries of Europe (the ARCAGE study) and including 1536 cases (749 oral cavity/pharynx, 574 larynx, 159 esophagus and 54 cases with an overlapping site) and 1443 controls.

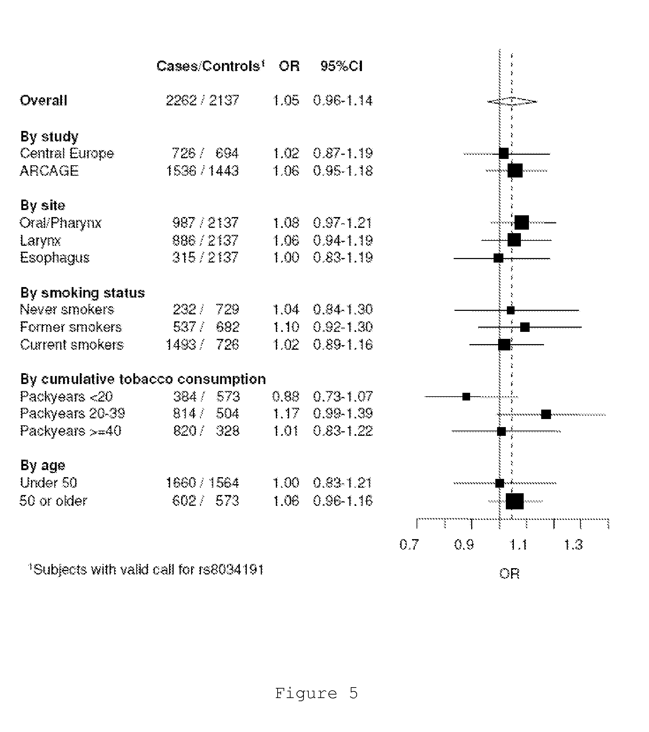

The figure 5 shows the OR and 95% CI for upper aero-digestive cancer for the rs8034191 variant using the co-dominant model among 2,262 cases and 2,137 controls. The ORs are standardized by age, sex and country.

The results show that no effect with rs8034191 was observed in either of the two studies separately or combined or in any of the cancer subgroups (See figure 5), implying that this association was specific for lung cancer.

### 7) Identity of the gene(s) associated with lung cancer predisposition

The present study has identified markers associated with lung cancer, said disease-associated markers spanning six known genes, including the nicotinic acetylcholine receptor subunits *CHRNA5, CHRNA3* and *CHRNB4,* the *IREB2* iron sensing response element, *PSMA4,* which is implicated in DNA repair, and a gene of unknown function designated *LOC123688* (see figure 2).

It was not possible to identify the most likely causal alleles or genes based on the differences in the strength of the statistical association because of the strong linkage disequilibrium.

However, the nicotinic acetylcholine receptor subunits are strong candidate genes. *CHRNA5* was the only gene found to contain a non-synonymous variant (rs16969968 in exon 5) with a strong disease association (p_{trend}=1.4x10⁻¹⁰). *CHRNA3* contained a synonymous variant in exon 5 of *CHRNA3* (rs1051730) that was also strongly associated with disease (p_{trend}=3x10⁻¹⁰).

While the other markers with strong disease-association either resided in introns or were inter-genic, it cannot be excluded that they could have a biological effect on one or more of the genes from the region.

However, other lines of evidence support a possible role for the nicotinic acetylcholine receptor subunit genes.

*CHRNA5, CHRNA3* and *CHRNB4* are part of a large family of nicotinic acetylcholine receptor subunit. These genes encode proteins forming receptors present in neuronal and other tissues that bind nicotine and nicotine derivatives. An association of *CHRNA3* and *CHRNA5* variants with nicotine dependence has been reported (SACCONE et al., abovementioned, 2007). The associated markers include the non-synonymous *CHRNA5* SNP, rs16969968, which is one of the markers of lung cancer risk. This SNP introduces a substitution of aspartic acid (D) to asparagine (N) at amino acid position 398 (D398N) of the CHRNA5 protein, located in the central part of the second intracellular loop. While the function of the second intracellular loop and the possible biological consequences of the D398N alteration, creating a charge change, remain to be elucidated, this amino acid is highly conserved across species suggesting that it could have functional importance. A T529A substitution in the second intracellular loop of *CHRNA4,* another nicotinic aceltycholine receptor subunit, is known to lead to altered responses to nicotine exposure in the mouse.

Within the ARCAGE study, all participants were asked a series of questions relating to tobacco addiction based on the FAGERSTROM tolerance questionnaire. No association between the genotyped marker of lung cancer risk, rs8034191, and any of the FAGERSTROM measures of nicotine addiction was observed, and similar results were observed for rs 16969968.

Consequently, nicotinic acetylcholine receptors may be involved in lung cancer through mechanisms other than tobacco dependence.

Finally, the expression of nicotine acetylcholine receptors can be inhibited by nicotine receptor antagonists that, if confirmed to be involved in disease etiology through such a mechanism, imply possible chemoprevention opportunities for lung cancer.

## Claims

1. A method of testing if a human, who is thought to be predisposed to having lung cancer, has or is predisposed to having lung cancer which comprises the steps of:
a) analyzing a biological sample from said human for detecting the presence of a single nucleotide polymorphism (SNP) associated with lung cancer, and
b) confirming if said human has or is predisposed for having lung cancer,
wherein said SNP associated with lung cancer is selected from the group comprising *rs17483548* (nucleotide N at position 16 of SEQ ID NO:1, wherein allele A is associated to lung cancer), *rs17405217* (nucleotide N at position 16 of SEQ ID NO:2, wherein allele T is associated to lung cancer), *rs17483721* (nucleotide N at position 16 of SEQ ID NO:3, wherein allele C is associated to lung cancer), *rs2656052* (nucleotide N at position 16 of SEQ ID NO:4, wherein allele G is associated to lung cancer), *rs2568494* (nucleotide N at position 16 of SEQ ID NO:5, wherein allele A is associated to lung cancer), *rs7181486* (nucleotide N at position 16 of SEQ ID NO:6, wherein allele C is associated to lung cancer), *rs17483929* (nucleotide N at position 16 of SEQ ID NO:7, wherein allele A is associated to lung cancer), *rs2656065* (nucleotide N at position 16 of SEQ ID NO:8, wherein allele T is associated to lung cancer), *rs2009746* (nucleotide N at position 16 of SEQ ID NO:9, wherein allele C is associated to lung cancer), *rs17484235* (nucleotide N at position 16 of SEQ ID NO:10, wherein allele G is associated to lung cancer), *rs1504550* (nucleotide N at position 16 of SEQ ID NO:11, wherein allele C is associated to lung cancer), *rs17484524* (nucleotide N at position 16 of SEQ ID NO:12, wherein allele G is associated to lung cancer), *rs9788721* (nucleotide N at position 16 of SEQ ID NO:13, wherein allele C is associated to lung cancer), *rs8034191* (nucleotide N at position 16 of SEQ ID NO:14, wherein allele C is associated to lung cancer), *rs10519203* (nucleotide N at position 16 of SEQ ID NO:15, wherein allele G is associated to lung cancer), *rs8031948* (nucleotide N at position 16 of SEQ ID NO:16, wherein allele T is associated to lung cancer), *rs931794* (nucleotide N at position 31 of SEQ ID NO:17, wherein allele G is associated to lung cancer), *rs2036527* (nucleotide N at position 31 of SEQ ID NO:18, wherein allele A is associated to lung cancer), *rs17486278* (nucleotide N at position 16 of SEQ ID NO:19, wherein allele C is associated to lung cancer), *rs7180002* (nucleotide N at position 16 of SEQ ID NO:20, wherein allele T is associated to lung cancer), *rs951266* (nucleotide N at position 16 of SEQ ID NO:21, wherein allele T is associated to lung cancer), *rs1317286* (nucleotide N at position 16 of SEQ ID NO:24, wherein allele G is associated to lung cancer), and *rs17487223* (nucleotide N at position 16 of SEQ ID NO:25, wherein allele T is associated to lung cancer).

2. The method according to claim 1, wherein said biological sample is a blood sample.

3. The method according to claim 1 or 2, wherein said single nucleotide polymorphism on chromosome 15q25 associated with lung cancer is selected from the group comprising *rs2656052* (nucleotide N at position 16 of SEQ ID NO:4, wherein allele G is associated to lung cancer), *rs17484235* (nucleotide N at position 16 of SEQ ID NO:10, wherein allele G is associated to lung cancer), *rs8034191* (nucleotide N at position 16 of SEQ ID NO:14, wherein allele C is associated to lung cancer), *rs10519203* (nucleotide N at position 16 of SEQ ID NO:15, wherein allele G is associated to lung cancer), *rs8031948* (nucleotide N at position 16 of SEQ ID NO:16, wherein allele T is associated to lung cancer), *rs931794* (nucleotide N at position 31 of SEQ ID NO:17, wherein allele G is associated to lung cancer), *rs2036527* (nucleotide N at position 31 of SEQ ID NO:18, wherein allele A is associated to lung cancer), and *rs1317286* (nucleotide N at position 16 of SEQ ID NO:24, wherein allele G is associated to lung cancer.

## Patentansprüche

1. Verfahren zum Prüfen, ob ein Mensch, von dem vermutet wird, dass er für Lungenkrebs prädisponiert ist, Lungenkrebs oder eine Prädisposition dafür hat, die folgenden Schritte aufweisend:
a) Analysieren einer biologischen Probe von dem Menschen, um nachzuweisen, ob ein mit Lungenkrebs assoziierter Einzelnukleotidpolymorphismus (SNP) vorhanden ist, und
b) Bestätigen, falls der Mensch Lungenkrebs oder eine Prädisposition dafür hat,
wobei der mit Lungenkrebs assoziierte SNP ausgewählt ist aus der Gruppe bestehend aus *rs17483548* (Nukleotid N an Position 16 von SEQ ID NO:1, bei dem Allel A mit Lungenkrebs assoziiert ist), *rs17405217* (Nukleotid N an Position 16 von SEQ ID NO:2, bei dem Allel T mit Lungenkrebs assoziiert ist), *rs17483721* (Nukleotid N an Position 16 von SEQ ID NO:3, bei dem Allel C mit Lungenkrebs assoziiert ist), *rs2656052* (Nukleotid N an Position 16 von SEQ ID NO:4, bei dem Allel G mit Lungenkrebs assoziiert ist), *rs2568494* (Nukleotid N an Position 16 von SEQ ID NO:5, bei dem Allel A mit Lungenkrebs assoziiert ist), *rs7181486* (Nukleotid N an Position 16 von SEQ ID NO:6, bei dem Allel C mit Lungenkrebs assoziiert ist), *rs17483929* (Nukleotid N an Position 16 von SEQ ID NO:7, bei dem Allel A mit Lungenkrebs assoziiert ist), *rs2656065* (Nukleotid N an Position 16 von SEQ ID NO:8, bei dem Allel T mit Lungenkrebs assoziiert ist), *rs2009746* (Nukleotid N an Position 16 von SEQ ID NO:9, bei dem Allel C mit Lungenkrebs assoziiert ist), *rs17484235* (Nukleotid N an Position 16 von SEQ ID NO: 10, bei dem Allel G mit Lungenkrebs assoziiert ist), *rs1504550* (Nukleotid N an Position 16 von SEQ ID NO: 11, bei dem Allel C mit Lungenkrebs assoziiert ist), *rs17484524* (Nukleotid N an Position 16 von SEQ ID NO: 12, bei dem Allel G mit Lungenkrebs assoziiert ist), *rs9788721* (Nukleotid N an Position 16 von SEQ ID NO: 13, bei dem Allel C mit Lungenkrebs assoziiert ist), *rs8034191* (Nukleotid N an Position 16 von SEQ ID NO: 14, bei dem Allel C mit Lungenkrebs assoziiert ist), *rs10519203* (Nukleotid N an Position 16 von SEQ ID NO: 15, bei dem Allel G mit Lungenkrebs assoziiert ist), *rs8031948* (Nukleotid N an Position 16 von SEQ ID NO: 16, bei dem Allel T mit Lungenkrebs assoziiert ist), *rs931794* (Nukleotid N an Position 31 von SEQ ID NO: 17, bei dem Allel G mit Lungenkrebs assoziiert ist), *rs2036527* (Nukleotid N an Position 31 von SEQ ID NO:18, bei dem Allel A mit Lungenkrebs assoziiert ist), *rs17486278* (Nukleotid N an Position 16 von SEQ ID NO: 19, bei dem Allel C mit Lungenkrebs assoziiert ist), *rs7180002* (Nukleotid N an Position 16 von SEQ ID NO:20, bei dem Allel T mit Lungenkrebs assoziiert ist), *rs951266* (Nukleotid N an Position 16 von SEQ ID NO:21, bei dem Allel T mit Lungenkrebs assoziiert ist), *rs1317286* (Nukleotid N an Position 16 von SEQ ID NO:24, bei dem Allel G mit Lungenkrebs assoziiert ist) und *rs17487223* (Nukleotid N an Position 16 von SEQ ID NO:25, bei dem Allel T mit Lungenkrebs assoziiert ist).

2. Verfahren nach Anspruch 1, wobei die biologische Probe eine Blutprobe ist.

3. Verfahren nach Anspruch 1 oder 2, wobei der Einzelnukleotidpolymorphismus auf Chromosom 15q25, der mit Lungenkrebs assoziiert ist, ausgewählt ist aus der Gruppe bestehend aus *rs2656052* (Nukleotid N an Position 16 von SEQ ID NO:4, bei dem Allel G mit Lungenkrebs assoziiert ist), *rs17484235* (Nukleotid N an Position 16 von SEQ ID NO: 10, bei dem Allel G mit Lungenkrebs assoziiert ist), *rs8034191* (Nukleotid N an Position 16 von SEQ ID NO: 14, bei dem Allel C mit Lungenkrebs assoziiert ist), *rs10519203* (Nukleotid N an Position 16 von SEQ ID NO: 15, bei dem Allel G mit Lungenkrebs assoziiert ist), *rs8031948* (Nukleotid N an Position 16 von SEQ ID NO: 16, bei dem Allel T mit Lungenkrebs assoziiert ist), *rs931794* (Nukleotid N an Position 31 von SEQ ID NO: 17, bei dem Allel G mit Lungenkrebs assoziiert ist), *rs2036527* (Nukleotid N an Position 31 von SEQ ID NO:18, bei dem Allel A mit Lungenkrebs assoziiert ist), und *rs1317286* (Nukleotid N an Position 16 von SEQ ID NO:24, bei dem Allel G mit Lungenkrebs assoziiert ist.

## Revendications

1. Méthode destinée à tester si un être humain, qui est supposé être prédisposé à développer un cancer du poumon, développe ou est prédisposé à développer un cancer du poumon qui comprend les étapes de :
a) analyse d'un échantillon biologique dudit être humain pour détecter la présence d'un polymorphisme mononucléotidique (SNP) associé au cancer du poumon, et
b) confirmation du fait que ledit humain développe ou est prédisposé à développer un cancer du poumon,
dans lequel ledit SNP associé au cancer du poumon est sélectionné dans le groupe comprenant *rs17483548* (nucléotide N en position 16 de SEQ ID NO : 1, dans lequel l'allèle A est associé au cancer du poumon), *rs17405217* (nucléotide N en position 16 de SEQ ID NO : 2, dans lequel l'allèle T est associé au cancer du poumon) , *rs17483721* (nucléotide N en position 16 de SEQ ID NO : 3, dans lequel l'allèle C est associé au cancer du poumon), *rs2656052* (nucléotide N en position 16 de SEQ ID NO : 4, dans lequel l'allèle G est associé au cancer du poumon), *rs2568494* (nucléotide N en position 16 de SEQ ID NO : 5, dans lequel l'allèle A est associé au cancer du poumon), *rs7181486* (nucléotide N en position 16 de SEQ ID NO : 6, dans lequel l'allèle C est associé au cancer du poumon), *rs17483929* (nucléotide N en position 16 de SEQ ID NO : 7, dans lequel l'allèle A est associé au cancer du poumon), *rs2656065* (nucléotide N en position 16 de SEQ ID NO : 8, dans lequel l'allèle T est associé au cancer du poumon), *rs2009746* (nucléotide N en position 16 de SEQ ID NO : 9, dans lequel l'allèle C est associé au cancer du poumon), *rs17484235* (nucléotide N en position 16 de SEQ ID NO : 10, dans lequel l'allèle G est associé au cancer du poumon), *rs1504550* (nucléotide N en position 16 de SEQ ID NO : 11, dans lequel l'allèle C est associé au cancer du poumon), *rs17484524* (nucléotide N en position 16 de SEQ ID NO : 12, dans lequel l'allèle G est associé au cancer du poumon), *rs9788721* (nucléotide N en position 16 de SEQ ID NO : 13, dans lequel l'allèle C est associé au cancer du poumon), *rs8034191* (nucléotide N en position 16 de SEQ ID NO : 14, dans lequel l'allèle C est associé au cancer du poumon), *rs10519203* (nucléotide N en position 16 de SEQ ID NO : 15, dans lequel l'allèle G est associé au cancer du poumon), *rs8031948* (nucléotide N en position 16 de SEQ ID NO : 16, dans lequel l'allèle T est associé au cancer du poumon), *rs931794* (nucléotide N en position 31 of SEQ ID NO : 17, dans lequel l'allèle G est associé au cancer du poumon), rs2036527 (nucléotide N en position 31 of SEQ ID NO : 18, dans lequel l'allèle A est associé au cancer du poumon), *rs17486278* (nucléotide N en position 16 de SEQ ID NO : 19, dans lequel l'allèle C est associé au cancer du poumon), *rs7180002* (nucléotide N en position 16 de SEQ ID NO : 20, dans lequel l'allèle T est associé au cancer du poumon), *rs951266* (nucléotide N en position 16 de SEQ ID NO : 21, dans lequel l'allèle T est associé au cancer du poumon), *rs1317286* (nucléotide N en position 16 de SEQ ID NO: 24, dans lequel l'allèle G est associé au cancer du poumon), et *rs17487223* (nucléotide N en position 16 de SEQ ID NO : 25, dans lequel l'allèle T est associé au cancer du poumon).

2. Méthode selon la revendication 1, dans laquelle ledit échantillon biologique est un échantillon de sang.

3. Méthode selon la revendication 1 ou 2, dans laquelle ledit polymorphisme mononucléotidique sur le chromosome 15q25 associé au cancer du poumon est sélectionné dans le groupe comprenant *rs2656052* (nucléotide N en position 16 de SEQ ID NO : 4, dans lequel l'allèle G est associé au cancer du poumon), *rs17484235* (nucléotide N en position 16 de SEQ ID NO : 10, dans lequel l'allèle G est associé au cancer du poumon), *rs8034191* (nucléotide N en position 16 de SEQ ID NO : 14, dans lequel l'allèle C est associé au cancer du poumon), *rs10519203* (nucléotide N en position 16 de SEQ ID NO : 15, dans lequel l'allèle G est associé au cancer du poumon), *rs8031948* (nucléotide N en position 16 de SEQ ID NO : 16, dans lequel l'allèle T est associé au cancer du poumon), *rs931794* (nucléotide N en position 31 of SEQ ID NO : 17, dans lequel l'allèle G est associé au cancer du poumon), *rs2036527* (nucléotide N en position 31 of SEQ ID NO : 18, dans lequel l'allèle A est associé au cancer du poumon), et *rs1317286* (nucléotide N en position 16 de SEQ ID NO : 24, dans lequel l'allèle G est associé au cancer du poumon.
